(19) Europäisches Patentamt
European Patent Office
Office européen des brevets

(11) Numéro de publication: **0 214 908**
**B1**

(12) **FASCICULE DE BREVET EUROPEEN**

(45) Date de publication du fascicule du brevet: **07.11.90**

(51) Int. Cl.$^5$: **C 07 H 21/00,** A 61 K 31/70

(21) Numéro de dépôt: **86401927.8**

(22) Date de dépôt: **02.09.86**

(54) **Nouveaux composés comportant une séquence d'oligonucléotide liée à un agent d'intercalation et à un groupement chimique activable, leur synthèse et leurs applications, à titre de nucléases artificielles spécifiques de séquences.**

(30) Priorité: **04.09.85 FR 8513132**

(43) Date de publication de la demande:
**18.03.87 Bulletin 87/12**

(45) Mention de la délivrance du brevet:
**07.11.90 Bulletin 90/45**

(84) Etats contractants désignés:
**AT BE CH DE FR GB IT LI LU NL SE**

(56) Documents cités:
**EP-A-0 117 777**

**TETRAHEDRON LETTERS, vol. 24, no. 14, 1983, pages 1523-1526, Pergamon Press Ltd., Oxford, GB; Y. HASHIMOTO et al.: "Synthesis of porphyrin(Fe)-intercalators which cause DNA scission"**

**JOURNAL OF THE CHEMICAL SOCIETY, CHEMICAL COMMUNICATIONS, 1982, pages 1298-1300, Londres, GB; J.W. LOWN et al.: "Bleomycin models. Haemin-acridines which bind to DNA and cause oxygen-dependent scission"**

(73) Titulaire: **CENTRE NATIONAL DE LA RECHERCHE SCIENTIFIQUE (CNRS)
15, Quai Anatole France
F-75007 Paris (FR)**

(72) Inventeur: **Thuong, Nguyen Thanh
31, route de Tigy - Vienne en Val
F-45510 Tigy (FR)**
Inventeur: **Helene, Claude
252, rue Haute
F-45590 Saint-Cyr en Val (FR)**

(74) Mandataire: **Warcoin, Jacques et al
Cabinet Régimbeau 26, avenue Kléber
F-75116 Paris (FR)**

(56) Documents cités:
**COMPTES RENDUS DE L'ACADEMIE DES SCIENCES PARIS, vol. 302, série II, no. 2, 1986, pages 75-80, Académie des Sciences, Paris, FR; M. BOIDOT-FORGET et al.: "Chimie organique biologique. - Nucléases artificielles: Coupure spécifique d'un acide nucléique par un oligodésoxynucléotide lié de façon covalente à l' EDTA et à un agent intercalant"**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 214 908 B1

**Description**

La présente invention concerne des composés chimiques nouveaux ainsi que leur application, notamment, à titre de nucléases artificielles spécifiques de séquences.

La présente invention concerne également l'application de ces composés pour le blocage sélectif de l'expression d'un gène.

Par gène on entend non seulement le gène endogène (oncogène pr exemple) mais également le gène exogène (ADN ou ARN de virus, de parasites ou de bactéries, par exemple).

Les composés chimiques selon la présente invention sont constitués par un oligonucléotide ou un oligodésoxynucléotide comportant un enchaînement de nucléotides naturels ou modifiés et lié par une liaison covalente à un radical intercalant et un radical chimique porteur d'au moins une fonction réagissant directement ou indirectement avec les chaînes de nucléotides (qui sera appelé ci-après "radical chimique réactif"), ce radical chimique réactif pouvant également constituer le radical intercalant.

Parmi ces composés, il faut citer plus particulièrement les composés de formule:

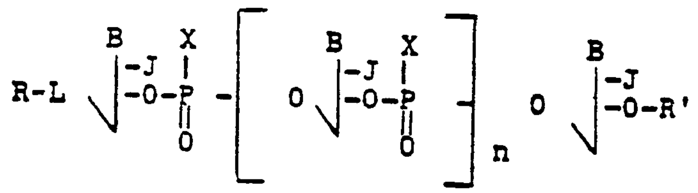

(A)

dans laquelle:

les radicaux B peuvent être identiques ou différents et representent chacun une base d'un acide nucléique, naturelle ou modifiée;

les radicaux X, qui peuvent être identiques ou différents, représentent chacun un oxoanion $O^{\ominus}$, un thioanion $S^{\ominus}$, un groupe alkyle, un groupe alcoxy, aryloxy, alcoxy ou alkyle substitué par un hétérocycle azoté, un groupe aminoalkyle, un groupe aminoalcoxy, un groupe thioalkyle ou un groupement —Y—Z;

R et R' peuvent être identiques ou différents et représentent chacun un groupement —Y—Z ou un groupement —Y'—Z' dans lesquels Y et Y' representent un radical alcoylène droit ou ramifié —alK—, ou des radicaux

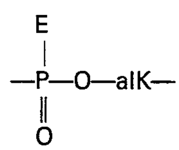 et 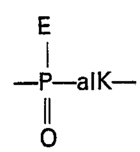

dans lesquels E peut avoir les mêmes significations que X, ou un radical —AlK—O—alK—, ou un radical

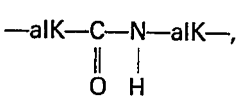, ou un radical 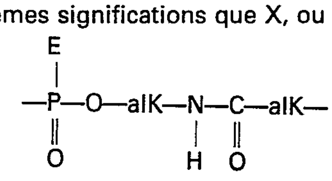 ou un radical 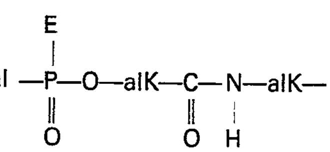

Z et Z' représentent chacun un radical intercalant ou un radical porteur d'au moins une fonction, réagissant directement ou indirectement avec les chaînes de nucléotides, l'un des R ou R' pouvant représenter H lorsque le radical chimique réactif est également un radical intercalant; sous réserve que le composé A comporte au moins un radical intercalant et un radical chimique réactif, dans le cas où Z ou Z' représente un radical intercalant qui est également un radical chimique réactif, alors Z et Z' peuvent être identiques ou bien l'un des R ou R' peut représenter l'hydrogène;

L représente un atome d'oxygène, un atome de soufre ou un groupe —NH—;

J représente un atome d'hydrogène ou un groupe hydroxy;

n est un nombre entier y compris O.

Dans la description, on utilise la représentation condensée des nucléotides suivante:

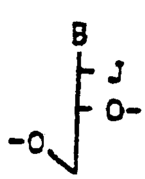

qui correspond à la formule développée:

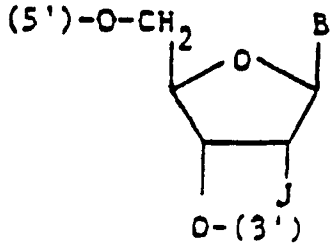

sur laquelle ont été mentionnées les extrémités (3′) et (5′).

Il convient de remarquer que la formule A représente un enchaînement de nucléotides qui peuvent être identiques ou différents, n indiquant simplement le nombre de nucléotides compris dans la molécule; n est de préférence un nombre compris entre 1 et 50, et de préférence entre 1 et 25.

Les radicaux intercalants correspondent à des agents d'intercalation qui sont des composés connus dans les techniques touchant aux acides nucléiques; il s'agit de composés capables de "s'intercaler" dans la structure des ADN ou des ARN.

Ces agents d'intercalation sont, en général, constitués par des composés polycycliques ayant une configuration plane tels que l'acridine, la furocoumarine, la daunomycine, la 1,10-phénanthroline, la phénanthridinium, les porphyrines, les dérivés de la dipyrido (1,2-a : 3′,2′-d) imidazole, l'ellipticine ou l'ellipticinium et leurs dérivés.

Les radicaux chimiques réactifs sont, de préférence, les radicaux chimiques de scission, c'est-à-dire que ces radicaux peuvent directement ou indirectement réagir pour scinder une chaîne de nucléotides. De préférence, ces radicaux chimiques réactifs seront activables, par exemple par voie chimique ou photochimique.

Les groupes réactifs de scission activables sont, par exemple, les dérivés de composés tels que:
l'acide éthylène-diamine-tétracétique
l'acide diéthylène-triamine-pentaacétique
les porphyrines,
la 1,10-phénanthroline, et
autres groupes aromatiques absorbant les radiations du proche U.V. et du visible.

Les groupements chimiquement activables en présence d'ions métalliques, d'oxygène et d'un agent réducteur (acide éthylène-diamine-tétracétique, acide diéthylène-triamine-penta-acétique, porphyrine, phénanthroline) induisent des coupures dans des séquences d'acides nucléiques situées dans leur voisinage.

Par irradiation dans le domaine visible ou ultraviolet, on peut activer les dérivés absorbant ces radiations et réaliser des réactions de photolyse des acides nucléiques sur lesquels est fixé l'oligonucléotide porteur du groupement photoactivable.

Parmi les radicaux Z et Z′, il faut citer en particulier:
les radicaux dérivés de l'EDTA

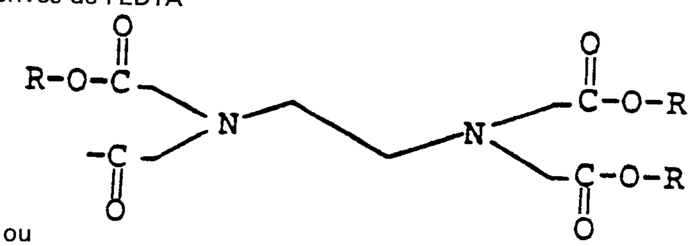

l'EDTA R = H, ou
$Me_3$ EDTA R = Me,
les radicaux dérivés de la méthylpyroporphyrine

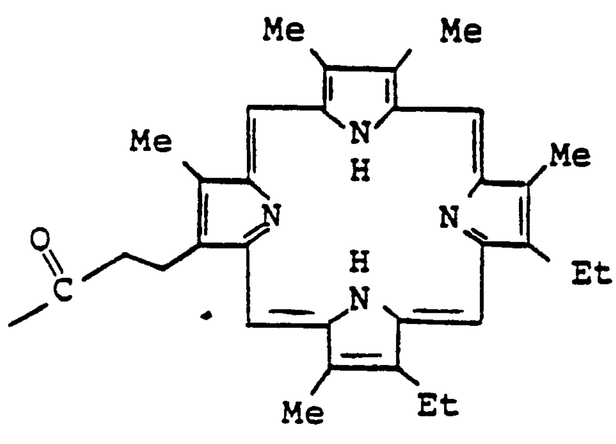

les radicaux dérivés de la phénanthroline

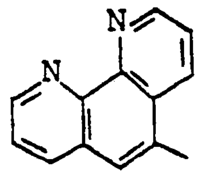

Le radical B est, de préférence, constitué par une base naturelle d'un acide nucléique, par exemple la thymine, l'adénine, la cytosine, la guanine ou l'uracile; mais, il est également possible d'utiliser des bases d'acides nucléiques modifiées, comme la 2-amino-adénine et ses dérivés substitués, par exemple sur le $N^6$ par un groupe aminoalkylène ou par un groupe azidophénylalkylène; ou la guanine substituée sur le $O^6$, par exemple par un groupe (ω-alkylène)-9-acridine; ou la 8-(ω-aminoalkyl)-amino-adénine et ses dérivés substitués sur le $NH_2$ en ω par un groupe acridine; ou des dérivés halogénés ou azidés, par exemple la 5-bromo-uracile ou la 8-azidoadénine. En outre, il est possible d'utiliser des dérivés photoactivables des bases.

Le radical X, bien qu'il représente de préférence un oxoanion, peut avoir d'autres significations; lorsque le radical X représente un groupement alkyle, il s'agit de préférence d'un groupement alkyle inférieur en $C_1$ à $C_7$ et, par exemple, les groupements éthyle, méthyle ou propyle; lorsque le radical X représente un groupe alcoxy, il s'agit de préférence d'un groupement alcoxy inférieur en $C_1$ à $C_7$, par exemple le groupement méthoxy ou éthoxy; lorsque X représente un groupement amino-alkyle ou aminoalcoxy, il peut s'agir d'un groupement amino-alkyle mono-substitué, disubstitué ou bien d'un radical amino sous forme de sel d'ammonium quaternaire. Dans ces conditions, les substituants sont, de préférence, des radicaux alkyles inférieurs comme définis précédemment; quant à la chaîne alkyle ou alcoxy reliant le radical amino au phosphore, il s'agit de préférence d'une chaîne droite ou ramifiée comportant de 1 à 10 atomes de carbone. Lorsque le radical alkyle ou alcoxy est substitué par un hétérocycle azoté, il s'agit notamment de cycle saturé à 5—6 chaînons comportant un atome d'azote qui peut être quaternaire. Enfin lorsque, le radical X est un radical thioalkyle, il s'agit de préférence d'un radical thioalkyle inférieur, c'est-à-dire qui comporte entre 1 et 7 atomes de carbone.

Les composés de la présente invention peuvent être préparés par voie chimique par des procédés déjà connus, en particulier par les procédés décrits dans les demandes de brevets français n° 83 01223 du 27 janvier 1983 et 84 11795 du 25 juillet 1984.

Selon la présente invention, on peut également préparer les dérivés totalement protégés et éliminer les groupements protecteurs.

Les composés de l'invention peuvent également être préparés par des méthodes semi-synthétiques, en particulier en utilisant des fragments d'ADN ou d'ARN préparés par des méthodes de génie génétique sur lesquels sont ensuite fixés un agent intercalant et un groupe chimique réactif.

La présente invention concerne également l'application des composés précédents sous forme de sel avec des bases ou des acides, et des composés sous forme racémique, ou sous forme d'isomères R ou S optiques purifiés ou en mélange.

Les composés selon la présente invention sont constitués d'une chaîne oligonucléotidique liée de façon covalente à un agent intercalant et à un groupe chimique réactif, ou à un agent intercalant qui est également un groupe chimique réactif. Dans ces nouvelles substances, l'oligonucléotide assure la reconnaissance de la séquence d'acide nucléique complémentaire, l'agent intercalant augmente fortement la stabilité du complexe d'hybridation et le groupe réactif conduit par activation à une hydrolyse de la chaîne d'acide nucléique complémentaire. Ce système permet donc de couper avec une grande efficacité une chaîne d'acides nucléiques en un site préalablement choisi.

Les composés de formule A de l'invention, par leur grande affinité spécifique pour les séquences nucléiques complémentaires, sont à la fois supérieurs aux oligonucléotides liés à un groupe éthylène-diamine-tétraacétique déjà connus (FEBS Letters, *172* (1984), 43—46; Proc. Nat. Acad. Sci. USA, *82* (1985), 968—972; Proc. Nat. Acad. Sci USA, *82* (1985), 963—967) dont l'affinité pour les séquences complémentaires est beaucoup plus faible (voir exemple VIII) et aux composés comportant un agent intercalant relié à un groupe éthylène-diamine-tétraacétique (Biochemistry, *23*, (1984), 3934—3945) ou à une porphyrine (J. Chem. Soc., Commun 1982), 1298—1300; Tetrahydron Letters, *24* (1983), 1523—1526) qui ne possèdent pas de spécificité vis-à-vis d'une séquence d'acides nucléiques donnée.

Les composés de la présente invention, par leur propriété de se fixer fortement sur la séquence nucléique complémentaire puis d'induire la coupure de la chaîne d'acide nucléique en ce site, sont utilisables à titre de nucléases artificielles spécifiques de séquences. Ils peuvent être utilisés comme réactifs en biologie moléculaire ou en génie génétique. Ils peuvent être également utilisés pour obtenir des fragments d'acides nucléiques, en particulier des fragments d'ARNs pour lesquels il n'existe pas de nucléase spécifique de séquences.

L'objet de la présente invention est également l'application des composés de formule A pour réaliser le blocage spécifique des gènes cellulaires ou d'agents pathogènes préalablement choisis (virus, bactéries, parasites).

Les composés selon l'invention sont donc utiles comme médicament afin de détruire sélectivement les gènes indésirables, exogènes (virus, bactéries, parasites) ou endogènes (gènes cellulaires oncogènes par exemple). L'expression de ces gènes peut être bloquée en agissant soit directement sur l'ADN ou l'ARN porteur de l'information génétique, soit sur l'ARN messager, copie du gène, en bloquant alors toute traduction par coupure de l'ARN messager choisi pour cible.

Ce blocage peut être réalise en utilisant un composé de formule A dont la séquence est complémentaire de celle d'une région non complexée d'un ADN ou d'un ARN, et en particulier d'un ARN messager. L'hybridation puis la coupure de l'ARN messager par ces substances empêchent la synthèse de la protéine correspondante.

Si cette protéine est vitale pour le virus ou pour la bactérie ou pour le parasite, alors les composés de formule A agiront comme substances antivirales ou comme antibiotiques ou comme produits antiparasitaires.

Si cette protéine n'est pas vitale pour l'organisme, on peut alors supprimer sélectivement ses effets. Dans ce cas, les composés de formule A agiront par exemple soit comme substances antitumorales, lorsque le gène visé (ou son ARN messager) code pour une protéine transformante, soit comme une substance qui permet de supprimer le caractère de résistance aux antibiotiques, aux antiviraux et aux antiparasitaires lorsque la protéine codée est responsable de l'inactivation de ces antibiotiques, de ces antiviraux et de ces antiparasitaires.

Les exemples ci-après destinés à illustrer d'autres caractéristiques et avantages de la présente invention.

Dans les exemples, les abréviations ci-après sont utilisées:
A 2'-déoxyadénosine
bzA N-benzoyl-2'-déoxyadénosine
ABH acide benzohydroxamique
Ar parachlorophényle
CNEt β-cyanoéthyle
DBU 1,8-diazabicyclo (5—4—0 undec-7-ène)
Me méthyle
DMTr diméthoxytrityle
MMTr monométhoxytrityle
MST mésitylènesulfonyltétrazolide
T thymidine

Dans les formules (DMTr) $(T \mp)_n$ T-Ar, la liaison phosphodiester est représentée par un trait et la liaison phosphotriester est représentée par le symbole (∓), chaque phosphate est protégé par le groupe p-chlorophényle (Ar).

Acr

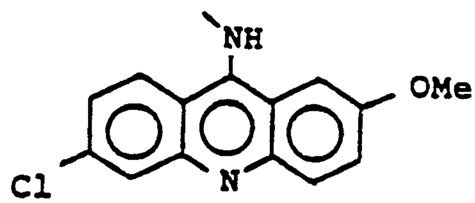

EDTA (R = H)

$Me_3$ EDTA (R = Me)

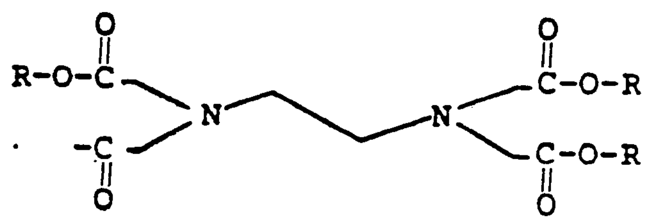

MPPo méthylpyrroporphyrine XXI

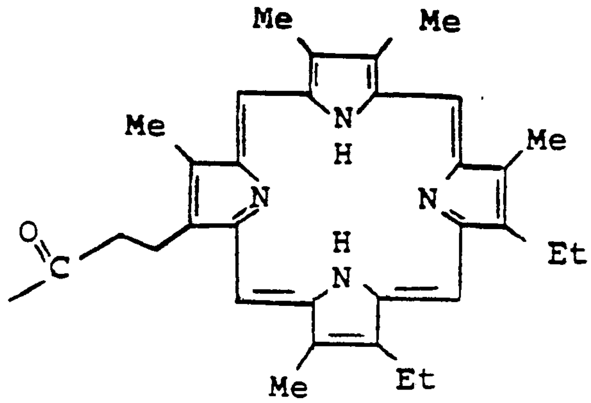

Phe

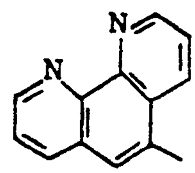

Exemple 1

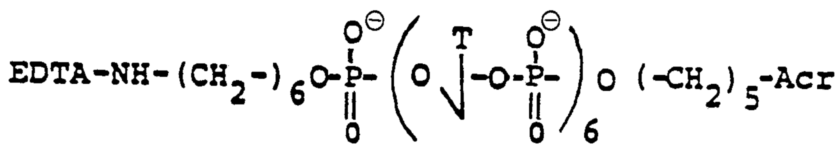

1) $Me_3EDTA—NH—(CH_2—)_6OH$

On fait réagir pendant 1 heure à 100°C sous atomosphère d'azote 1 équivalent d'ester tétraméthylique d'acide éthylène-diamine-tétraacétique, 1 équivalent d'amino-6-hexanol-1 et 0,5 équivalent d'hydroxy-2-pyridine puis on purifie le produit obtenu par chromatographie sur silice en utilisant le système de solvant $CH_2Cl_2$, MeOH (98:2 à 95:5, v/v). Rendement:environ 40%.

2) $Ar—(T\mp)_6(CH_2)_5Acr$

On ajoute sous-agitation, et à −20°C 3 équivalents de méthyl-p-chlorophényl-chlorophosphate dans la pyridine anhydre; l'addition terminée, on continue l'agitation pendant 1 heure à la température ambiante, puis on ajoute à −10°C une solution de 1 équivalent d'hexanucléotide $(T\mp)_6$ $(CH_2)_5Acr$ (préparé selon le brevet français n° 83 01223). Après 1 heure de réaction à la température ambiante, on ajoute de l'eau glacée puis on extrait le composé $Ar—(T\mp)_6—(CH_2)_5Acr$ avec du dichlorométhane. Le rendement de la préparation est pratiquement quantitatif.

3) 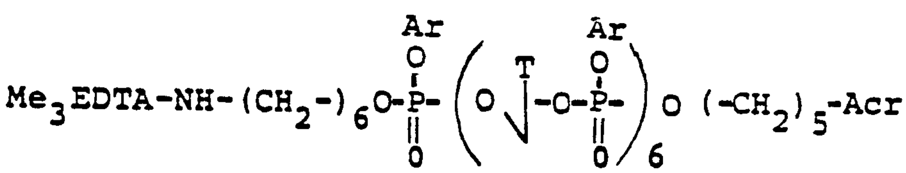

A une solution de 3 équivalents de $Me_3EDTA—NH—(CH_2—)_6OH$ et de 1 équivalent de $AR—(T\mp)_6(CH_2)_5Acr$ dans la pyridine anhydre, on ajoute à la température ambiante et sous agitation 2,5-équivalents de mésitylène-sulfonyltétrazolide (MST). On continue l'agitation pendant 1 heure à la température ambiante puis on détruit l'excès de réactif de couplage par addition d'eau glacée, le produit est extrait avec du dichlorométhane puis purifié sur gel de silice en utilisant le système de solvant $CH_2Cl_2$, MeOH (85:15, v/v).

4) L'hexanucléotide totalement protégé préparé selon l'exemple I—3 est traité pendant 24 heures sous agitation et à la température ambiante avec une solution molaire en acide benzohydroxamique (ABH) et en 1,8-diazabicyclo-(5—4—O undec-7-ène) (DBU) dans la pyridine anhydre et en utilisant 10 équivalents de

ABH—DBU par équivalent de phosphoester arylé à déprotéger; à ce mélange reactionnel, on ajoute ensuite 2 volumes de solution de soude 0,7 M puis on continue l'agitation pendant 4 heures à température ambiante. Après avoir neutralisé le milieu réactionnel avec l'acide chlorhydrique, on lave la phase aqueuse avec de l'éther puis on purifie l'oligonucléotide déprotégé par chromatographie liquide d'abord par échange d' ion puis en phase inverse. Colonne de polyanion HR 5/5 (Pharmacia), débit 1 ml/mn, solvant NaCl $10^{-2}$ M et NaCl 1,5 M, gradient linéaire de 0 à 100% en NaCl 1,5 M en 20 minutes, temps de rétention: 11 minutes 42 secondes. Le temps de rétention sur colonne $C_{18}$ est donné par le tableau I.

Exemple II

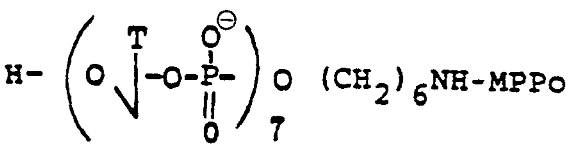

1) MPPo—NH—$(CH_2—)_6$O—H

On fait réagir pendant 5 heures à 100°C sous atmosphère d'azote et à l'abri de la lumière 1 équivalent d'ester éthylique de la méthylpyrroporphyrine XXI, 10 équivalents d'amino-6-hexanol-1 et 2 équivalents d'hydroxy-2-pyridine dans la pyridine. On élimine la pyridine sous vide, on reprend le résidu avec du chlorure de méthylène puis on acidifie avec HClN; la phase organique est lavée à l'eau puis évaporée sous vide. Le produit est ensuite purifié par chromatographie sur gel de silice en utilisant le système de solvant $CH_2Cl_2$, MeOH (98:2 à 95:5, v/v). Rendement: 70%.

2) DMTr $(T\mp)_7(CH_2)_6$NH MPPo

On opère comme dans l'exemple I—3 et en utilisant 1 équivalent de DMTr $(T\mp)_6$T—AR (préparé selon le brevet français 83 01223), 1,5 équivalents de MPPoNH$(CH_2)_6$OH et 3 équivalents de MST, on obtient l'heptanucléotide totalement protégé qui est ensuite purifié par chromatographie sur silice en utilisant le système de solvant $CH_2Cl_2$, $H_2O$, acétone (54:1:45 à 38:2:60 v/v). Rendement: 60%.

3) Un équivalent d'heptanucléotide protégé préparé selon l'exemple II—2 est traité avec 70 équivalents de ABH—DBU (solution molaire) pendant 24 heures à la température ambiante; le milieu réactionnel est neutralisé avec du DOWEX 50 (forme pyridinium) puis évaporé sous vide. Le résidu est repris avec l'acide acétique à 80% puis la solution est laissée 2 heures à la température ambiante.

On élimine l'acide acétique sous vide par évaporation plusieurs fois avec de l'éthanol; on reprend le résidu avec de l'eau puis on lave la phase aqueuse avec de l'éther. Le produit déprotégé est purifié par chromatographie liquide par échange d'ion puis en phase inverse; le tableau I donne le temps de rétention du produit obtenu.

Exemple III

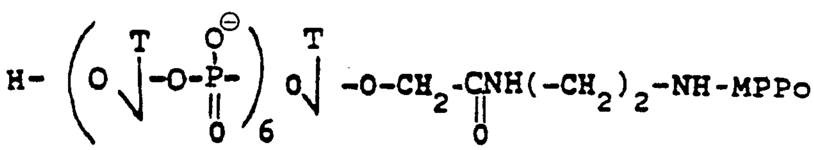

1) 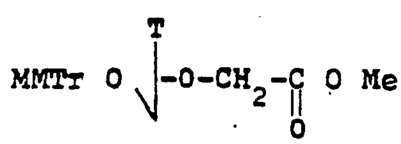

On fait réagir pendant 1 heure à la température ambiante 1 équivalent de 5'-monométhoxytritylthymidine, 1,1 équivalent de bromoacétate de méthyle et de 2,5 équivalents de DBU dans l'acétonitrile anhydre. Après avoir évaporé le solvant sous vide, on reprend le résidu avec du chloroforme puis on lave la phase chloroformique avec une solution de NaH $CO_3$ à 50%. On classe le solvant sous vide et on purifie le produit par chromatographie sur silice en utilisant le système de solvant $CH_2Cl_2$, MeOH (98:2 à 95:5, v/v). Rendement: 90%.

2) 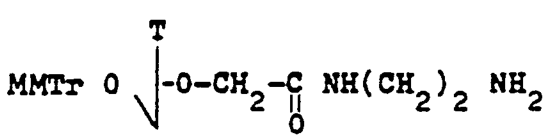

On chauffe sous atmosphère d'azote à 100°C pendant 1,30 heure un mélange d'un équivalent de composé préparé selon l'exemple III—1 de 5 équivalents d'éthylènediamine, de équivalents d'hydroxy-2-pyridine dans la pyridine anhydre. On chasse les produits volatils sous vide puis on purifie le produit par chromatographie sur gel de silice en utilisant le système de solvant MeOH, $NH_4OH$ concentré (100:0 à 95:5, v/v). Rendement 80%.

3) 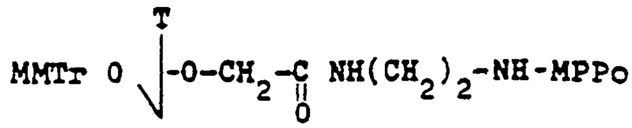

On fait réagir pendant 24 heures à la température ambiante 1,3 équivalents de composé prépare selon l'exemple III—2, 1 équivalent de sel de pyridinium de la méthylpyrroporphyrine XXI (préparé par hydrolyse d'ester éthylique de la méthylpyrroporphyrine XXI), 10 équivalents de dicyclohexylcarbodiimide dans la pyridine anhydre. On détruit l'excès de dicyclohexylcarbodiimide par addition d'eau, on filtre le précipité, on extrait le produit avec du chloroforme puis on le purifie par chromatographie sur silice en utilisant le système de solvant $CH_2Cl_2$, MeOH (98:2 à 94:6, v/v). Rendement: 90%.

4) 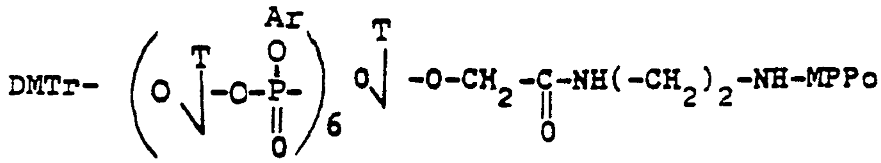

Un équivalent de composé préparé selon l'exemple III—3 est traité avec l'acide benzène sulfonique (2% en solution dans le chloroforme, méthanol (7:3, v/v)) à 0°C pendant 30 minutes à 1 heure. Le mélange est repris avec du chloroforme et lavé avec une solution aqueuse de $NaHCO_3$ à 5% puis séché sur $Na_2SO_4$ et concentré sous vide. A ce résidu on ajoute un équivalent de $DMTr(T\mp)_5T$—Ar puis on élimine les traces d'eau par évaporation 3 fois avec la pyridine. On ajoute à cette solution 2,5 équivalents de MST puis on termine la préparation comme dans l'exemple I—3 en utilisant le systèmes de solvant $CH_2Cl_2$, $H_2O$, acétone (54:1:45; 46:2:52 et 38:2:60, v/v) pour la chromatographie sur gel de silice.

5) On opére comme dans l'exemple II—3 en utilisant 60 équivalents de solution de ABH—DBU, on obtient l'oligonucléotide déprotégé dont le temps de rétention est donné par le tableau I.

Exemple IV

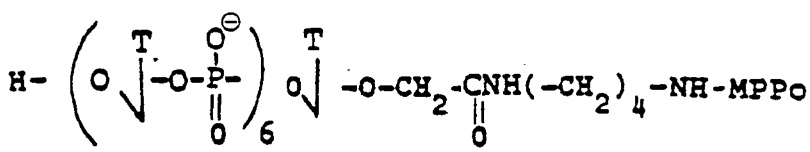

On opère comme dans l'exemple III en remplaçant l'éthylène diamine par la 1,4-diaminobutane, on obtient l'oligonucléotide dont le temps de rétention est donné par le tableau I.

Exemple V

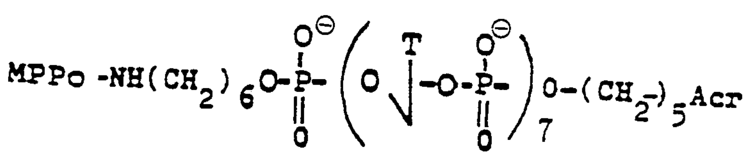

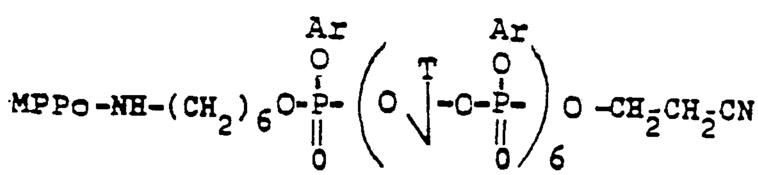

On fait réagir pendant 1 heure, sous agitation et à la température ambiante, 1 équivalent de dérivé de la méthylpyrroporphyrine XXI (préparé selon l'exemple II—1), 1 équivalent d'hexanucléotide

Ar—(T∓)$_6$CH$_2$CH$_2$CN (préparé selon le brevet français n° 83 01223) et 3 équivalents de MST dans la pyridine anhydre. Après avoir détruit l'excès de MST par addition d'eau glacée, le produit est extrait avec du chloroforme puis purifié sur gel de silice en utilisant le système de solvant $CH_2Cl_2$, $H_2O$, acétone (54:1:45 et 46:2:52, v/v).

2) 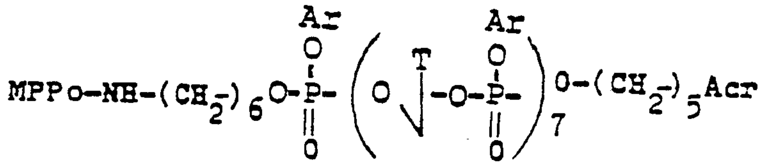

Le composé de l'exemple V—1 (1 équivalent) est traité pendant 2 heures à la température ambiante avec une solution de pyridine-triéthylamine (2:1, v/v); on chasse le solvant sous vide puis on lave le solide obtenu avec de l'éther. A ce produit on ajoute 1 équivalent de T∓(CH$_2$)$_5$Acr (préparé selon le brevet français n° 83 01223); après avoir séché le mélange par évaporation avec la pyridine, on ajoute à cette solution 2,5 équivalents de MST et on termine la préparation comme dans l'exemple I—3 en purifiant le produit par chromatographie sur gel de silice (plaque préparative, solvant $CH_2Cl_2$, MeOH (85:15, v/v)).

3) L'heptanucléotide protégé (1 équivalent) préparé d'après l'exemple V—2 est traité pendant 40 heures à la température ambiante avec une solution moléculaire en ABH—DBU (70 équivalents). On neutralise le milieu réactionnel avec le DOWEX 50 (forme pyridinium), on filtre et on chasse le solvant sous vide; le résidu est repris avec de l'eau, la phase aqueuse est lavée avec de l'éther. Le produit est ensuite purifié par chromatographie liquide par échange d'ion puis en phase inverse, le tableau I donne le temps de rétention de l'oligonucléotide préparé.

Exemple VI

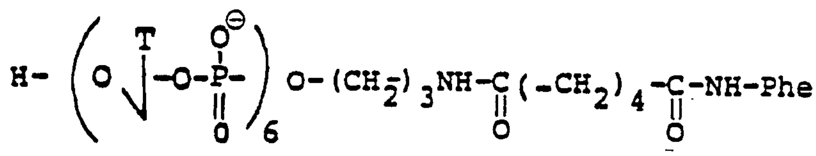

En utilisant 1,3 équivalents de DMTr(T∓)$_5$T—Ar, 1 équivalent de

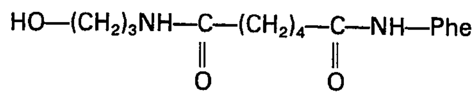

et 3 équivalents de MST et en opérant comme dans l'exemple I—3, on obtient l'hexanucléotide protégé:

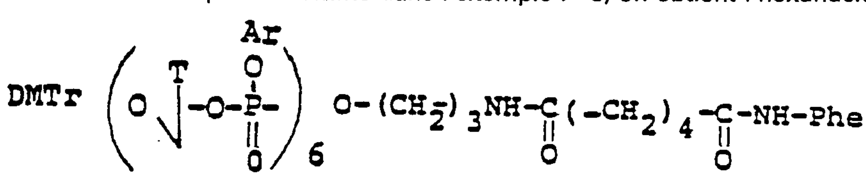

qui est ensuite déprotégé puis purifié selon l'exemple II—3. Le temps de rétention du produit purifié est donné par le tableau I.

Exemple VII

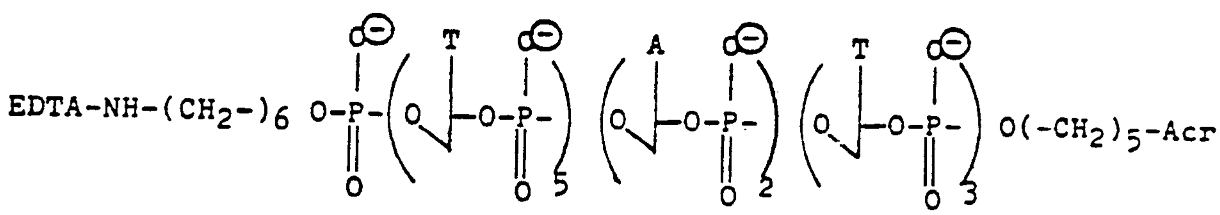

1) dT ∓ T ∓ T ∓ T ∓ T ∓ bzA ∓ bzA ∓ T ∓ T ∓ T ∓ (CH$_2$)$_5$Acr

Ce composé est préparé selon le brevet français n° 83 01223 en utilisant le schéma de réaction suivant:

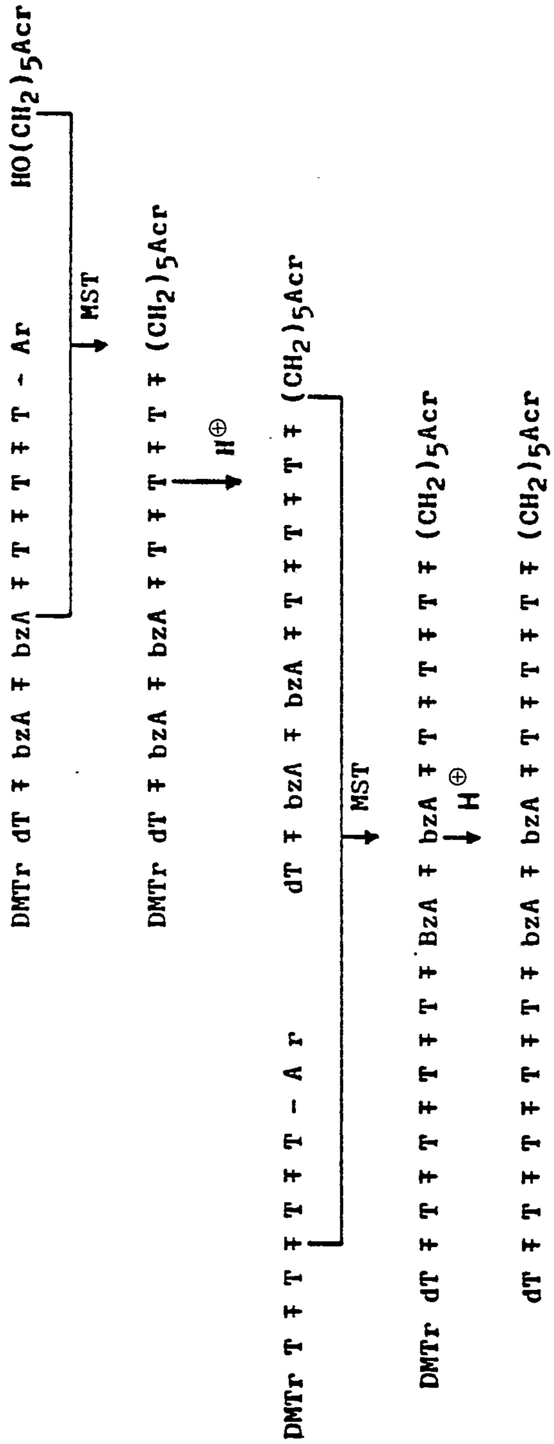


2)

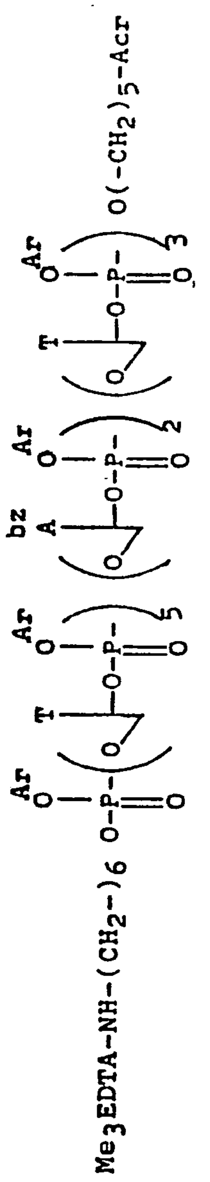

On opère comme dans les exemples I—2 et I—3 et en remplaçant les composés:

$(T\mp)_6(CH_2)_5Acr)$ et $Ar—(T\mp)_6(CH_2)_5Acr$

respectivement par les composés:

$d(T\mp)_5(bzA\mp)_2(T\mp)_3(CH_2)_5$ Acr et $dAr—(T\mp)_5(bzA\mp)_2(T\mp)_3(CH_2)_5Acr$

on a obtenu le décamère totalement protégé.
Chromatographie sur couche mince: gel de silice 60F254; solvant $CH_2Cl_2$, MeOH (9:1, v/v). Rf environ 0,1.

3) En partant du décamère totalement protégé préparé précédemment (exemple VII—2) et en opérant selon l'exemple I—4, on a obtenu le décamère déprotégé dont les temps de rétention en HPLC sont les suivants:

Colonne de polyanion HR 5/5 (Pharmacia), débit 1 ml/mn, solvant NaCl $10^{-2}$ M et NaCl 2 M, gradient linéaire de 0 à 100% en NaCl 2 M en 12 minutes, temps de rétention: 10 minutes 8 secondes.

Colonne Lichrosorbe RP—18 (Merck), débit 1,2 ml/mn solvant $CH_3CN$, acétate de triéthylammonium à 10% en poids dans l'eau, eau (15,8:9,47:74,72, v/v) (pH 5,9), isocratique, temps de rétention: 3 minutes 51 secondes.

TABLEAU I

| Exemple | Système de solvants | Temps de rétention |
|---|---|---|
| I | A—B, isocratique avec 40% de B | 2 mn 44 sec |
| II | A—B, isocratique avec 95% de B | 6 mn 30 sec |
| III | A—B, isocratique avec 95% de B | 3 mn 24 sec |
| IV | A—B, isocratique avec 95% de B | 4 mn 54 sec |
| V | A—B, isocratique avec 75% de B | 4 mn 04 sec |
| VI | A—B', isocratique avec 80% de B' | 2 mn 04 sec |

HPLC, colonne Lichrosorbe RP—18 (Merck); débit 1,2 ml/mn
A: $CH_3CN$, AAc, eau (2:10:88, v/v) (pH=5,9)
B: $CH_3CN$, AAc, eau (48:10:42, v/v) (pH=5,9)
B': $CH_3CN$, AAc, eau (24:10:66, v/v) (pH=5,9)
AAc: solution aqueuse d'acétate d'ammonium à 10% en poids (pH = 5,9)

Exemple VIII

Coupure spécifique du poly(dA) par le composé:

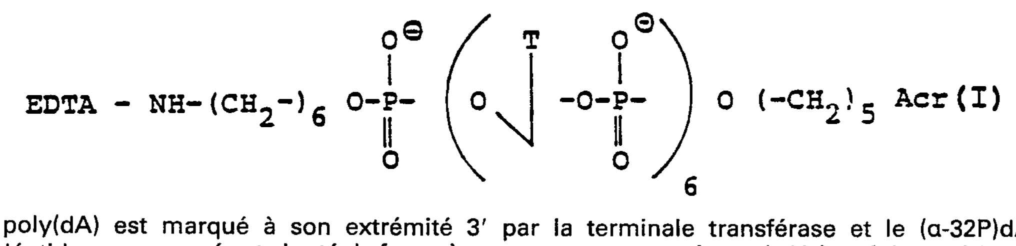

Le poly(dA) est marqué à son extrémité 3' par la terminale transférase et le (α-32P)dATP. Du polynucléotide non marqué est ajouté de façon à ce que sa concentration soit 10 à 50 fois supérieure à celle du polynucléotide marqué. Les concentrations indiquées sont celles du polynucléotide non marqué.

Une solution fraîche de $Fe(NH)_2(SO_4)_2$ est ajoutée ainsi que de l'eau oxygénée, du tampon Tris pH 8,3 et du chlorure de sodium. Des aliquots sont répartis dans des tubes où les oligonucléotides ont été lyophilisés et le volume total est complète à 4,5 µl. La réaction de coupure est déclenchée par l'addition de 0,5 µl dithiothréitol (DDT) 50 mM. Les concentrations finales sont [A] = 50 µM, [EDTA—Fe] = 20 µM, [NaCl] = 160 mM, [Tris] (pH 8,3) = 8 mM, $[H_2O_2]$ = 1 mM, [DTT] = 5 mM. Les réactions de coupure sont arrêtées par addition de 2 µl de ter-butanol qui capte les radicaux hydroxyles, intermédiaire de la réaction de coupure. Les échantillons sont lyophilisés, resuspendus dans 3 µl de formamide et appliqués sur gel de séquence à 8% en acrylamide (1/20 réticulation) et 50% en urée.

La durée de l'électrophorèse est 2 heures 30 minutes (sous 1300 V). Le gel est ensuite autoradiographié à 20°C avec un film Kodak®.

La figure 1 représente des résultats d'électrophorèse sur gel de polyacrylamide et montre l'effet d'une incubation du poly(dA) en présence de différents additifs à 19°C pendant 140 minutes. Les concentrations utilisées sont indiquées précédemment (A: $H_2O_2$ + Fe(II); B: $H_2O_2$ + Fe (II) + DTT; C: $H_2O_2$ + Fe(II) + DTT + EDTA (acide éthylènediaminetétraacétique); D: $H_2O_2$ + Fe (II) + DTT + $(TP)_7(CH_2)_6$NH—EDTA; E: $H_2O_2$ + Fe (II) + DTT + EDTA—$NH(CH_2)_6$ $p(Tp)_6$ $(CH_2)_5$Acr).

L'oligonucléotide EDTA—$NH(CH_2)_6$— $p(Tp)_6(CH_2)_5$Acr hydrolyse très efficacement le poly(dA) comme l'indiquement la disparition de la bande principale due au poly (dA) intact et l'apparition de bandes correspondant à des fragments de taille variable (y compris des fragments très courts). L'oligonucléotide $(Tp)_7(CH_2)_6$NH—EDTA possède une efficacité beaucoup plus faible. L'acide éthylènetétraacétique seul n'a pratiquement aucun effet sur le poly(dA) dans les mêmes conditions. Si le poly(dA) est remplacé par du poly(dT), aucune hydrolyse n'est observée en présence de EDTA—$NH(CH_2)_6p(Tp)_6(CH_2)_5$Acr. Cette expérience démontre que la réaction est spécifique de la séquence complémentaire de l'hexathymidylate.

Exemple IX

Coupure spécifique du poly(dA) par le composé de l'exemple VI

Le poly(dA) marqué est préparé comme indiqué à l'exemple VIII. Il est incubé à 20°C (concentration 19 μM) en présence du composé VI (5 μM), de phénanthroline (5 μM), de Cu(II) (5 μM) et de dithiothréitol (1 mM) dans un tampon phosphate (50 mM) contenant 0,1 M NaCl. Le poly (dA) est dégradé sélectivement dans ces conditions. Aucun effet n'est observé sur le poly(dT) ou la double hélice poly(dA). poly(dT). Aucune coupure n'est observée en l'absence de l'un des constituants du mélange réactionnel. Si le composé VI est remplacé par de la phénanthroline libre à la même concentration (5 μM) aucune coupure n'est observée pour le poly(dA), les doubles hélices poly(dA).oligo$(dT)_8$ et poly(dA).poly(dT).

**Revendications pour les Etats contractants: BE CH DE FR GB IT LI LU NL SE**

1. Composés chimiques constitués par un oligonucléotide ou un oligodésoxynucléotide comportant un enchaînement de nucléotides naturels ou modifiés et lié par une liaison covalente à un radical intercalant et à un radical chimique réactif ou à un radical intercalant qui est également un radical chimique réactif.

2. Composés selon la revendication 1 de formule:

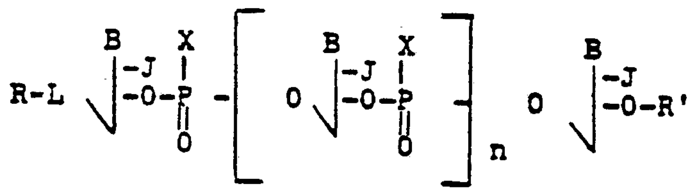

(A)

dans laquelle:

les radicaux B peuvent être identiques ou différents et representent chacun une base d'un acide nucléique, naturelle ou modifiée;

les radicaux X, qui peuvent être identiques ou différentes, représentent chacun un oxoanion $O^{\ominus}$, un thioanion $S^{\ominus}$, un groupe alkyle, un groupe alcoxy, aryloxy, alcoxy ou alkyle substitué par un hétérocycle azoté, un groupe aminoalkyle, un groupe aminoalcoxy, un groupe thioalkyle ou un groupement —Y—Z;

R et R' peuvent être identiques ou différents et représentent chacun un groupement —Y—Z ou un groupement —Y'—Z' dans lesquels Y et Y' representent un radical alcoylène droit ou ramifié —alK—, ou des radicaux

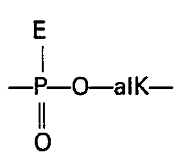 et 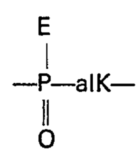

dans lesquels E peut avoir les mêmes significations que X, ou un radical —alK—O—alK—, ou un radical

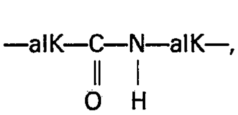, ou un radical 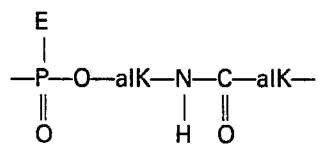 ou un radical 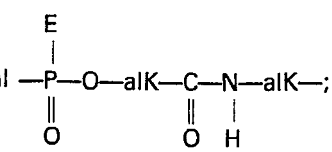;

Z et Z' représentent chacun un radical intercalant ou un radical porteur d'au moins une fonction, réagissant directement ou indirectement avec les chaînes de nucléotides, l'un des R ou R' pouvant représenter H lorsque le radical chimique réactif est également un radical intercalant; sous réserve que le composé A comporte au moins un radical intercalant et un radical chimique réactif, dans le cas où Z ou Z' représente

un radical intercalant qui est également un radical chimique réactif, alors Z et Z' peuvent être identiques ou bien l'un des R ou R' peut représenter l'hydrogène;

L représente un atome d'oxygène, un atome de soufre ou un groupe —NH—;

J représente un atome d'hydrogène ou un groupe hydroxy;

n est un nombre entier y compris O.

3. Composés selon la revendication 2, caractérisés en ce que le radical intercalant est dérivé des composés polycycliques ayant une configuration plane.

4. Composés selon la revendication 3, caractérisés en ce que le radical intercalant est dérivé de l'acridine, la furocoumarine, la daunomycine, la 10-phénanthroline, le phénanthridinium, les porphyrines, les dérivés de la dipyrido(1,2-a: 3',2'-d) imidazole, l'ellipticine ou l'ellipticinium et les dérivés de ces agents intercalants.

5. Composés selon les revendications 1 à 4, caractérisés en ce que les groupes chimiques réactifs sont choisis parmi les dérivés de l'acide éthylènediaminetétraacétique, de l'acide diéthylènetriamine-pentaacétique, des porphyrines, de la 1,10-phénanthroline.

6. Composés selon l'une des revendications 1 à 5, caractérisés en ce que le radical B est un radical correspondant à la thymine, l'adénine, la 2-aminoadénine et ses dérivés substitués, par exemple sur le $N^6$ par un groupe aminoalkylène ou par un groupe azidophénylalkylène, la cytosine, la guanine ou la guanine substituée sur le $O^6$, par exemple par un groupe (ω-alkylène)-9-acridine; ou la 8-(ω-aminoalkyl)-amino-adénine et ses dérivés substitués sur le $NH_2$ en ω par un groupe acridine; l'uracile, le 5-bromo-uracile, le 8-azido-adénine ou un dérivé photoactivable des bases.

7. Composés selon la revendication 1 de formule choisie parmi:

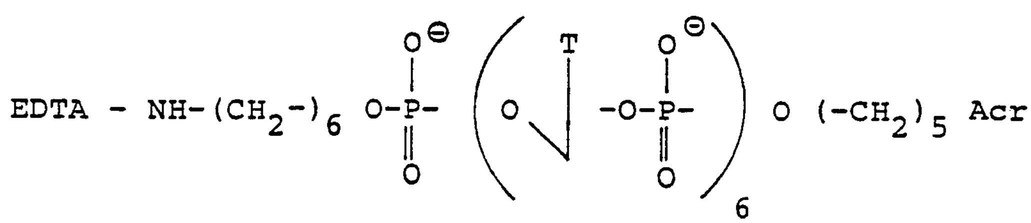

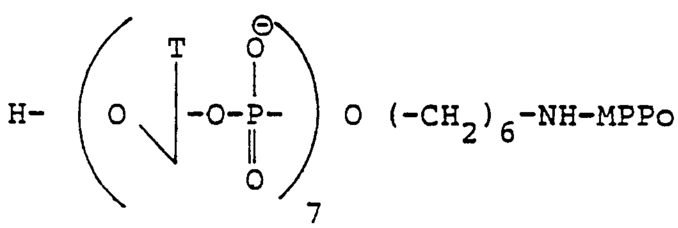

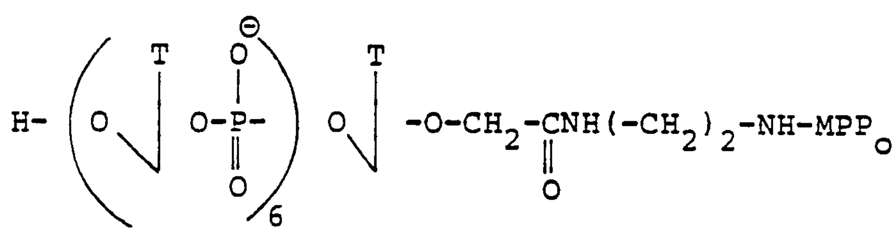

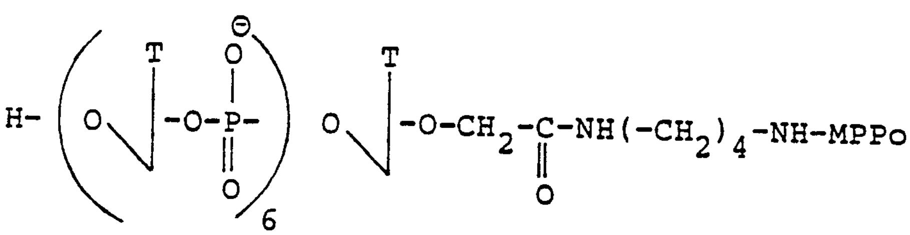

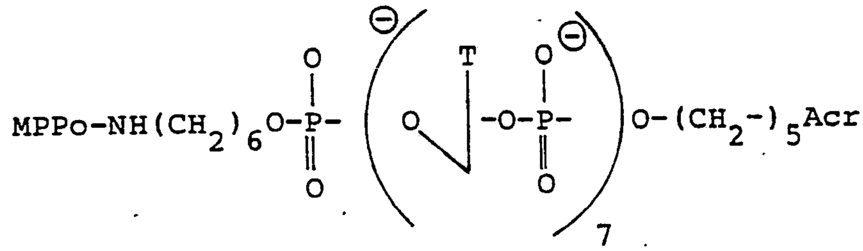

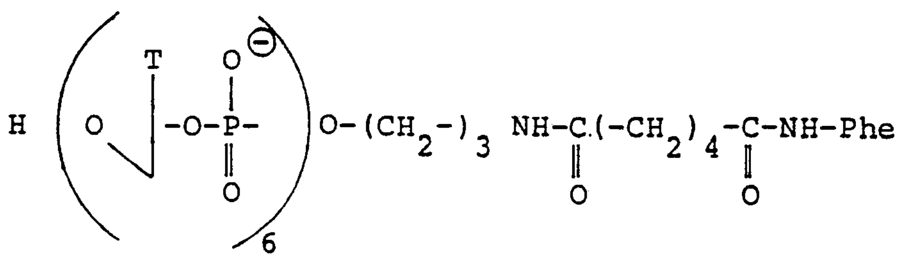

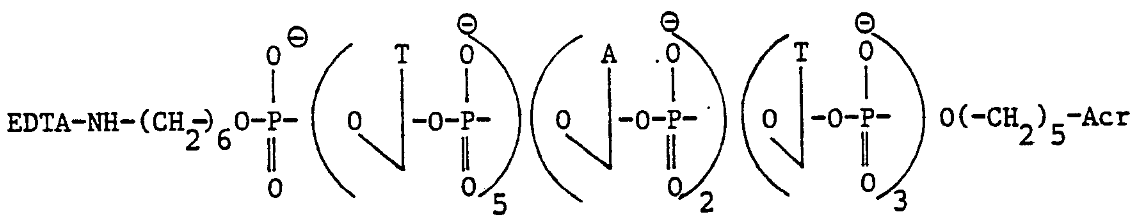

8. Procédé de préparation de composés selon l'une des revendications 1 à 7, caractérisé en ce que l'on prépare les dérivés totalement protégés et que l'on élimine les groupements protecteurs.

9. Application des composés selon l'une des revendications 1 à 7 à titre de nucléases artificielles spécifiques de séquences d'acides ribo et désoxyribonucléiques.

10. Application des composés selon l'une des revendications 1 à 7 pour obtenir des fragments d'acides nucléiques déterminés à partir d'acides ribo et désoxyribonucléiques.

11. Application des composés selon l'une des revendications 1 à 7 comme réactifs pour analyser les structures secondaires et tertiaires d'acides ribo et desoxyribonucléiques, par coupures sélectives de la séquence cible et des séquences voisines dans l'espace.

12. Application des composés selon l'une des revendications 1 à 7 pour couper et dégrader sélectivement les acides nucléiques en particulier les ARN messagers in vivo.

13. Application selon la revendication 12, pour bloquer sélectivement l'expression d'un gène par coupure sélective du gène ou de son ARN messager.

14. A titre de médicament, un composé selon l'une des revendications 1 à 7.

**Revendications pour l'Etat contractant: AT**

1. Procédé de préparation de composés chimiques constitués par un oligonucléotide ou un oligodésoxynucléotide comportant un enchaînement de nucléotides naturels ou modifiés et lié par une liaison covalente à un radical intercalant et à un radical chimique réactif ou à un radical intercalant qui est également un radical chimique réactif, caractérisé en ce que l'on prépare des dérivés totalement protégés et que l'on élimine les groupements protecteurs.

2. Procédé de préparation de composés selon la revendication 1 de formule:

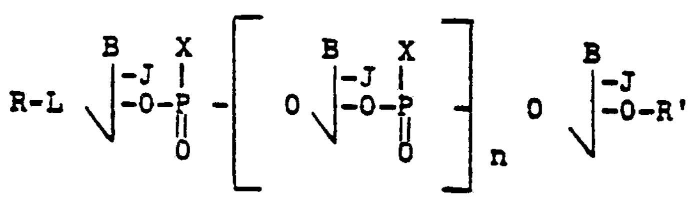

(A)

dans laquelle:

les radicaux B peuvent être identiques ou différents et représentent chacun une base d'un acide nucléique, naturelle ou modifiée;

les radicaux X, qui peuvent être identiques ou différents, représentent chacun un oxoanion $O^{\ominus}$, un thioanion $S^{\ominus}$, un groupe alkyle, un groupe alcoxy, aryloxy, alcoxy ou alkyle substitué par un hétérocycle azoté, un groupe aminoalkyle, un groupe aminoalcoxy, un groupe thioalkyle ou un groupement —Y—Z;

R et R′ peuvent être identiques ou différents et représentent chacun un groupement —Y—Z ou un groupement —Y′—Z′ dans lesquels Y et Y′ représentent un radical alcoylène droit ou ramifié —alK—, ou des radicaux

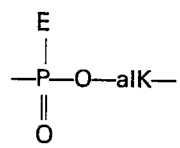 et 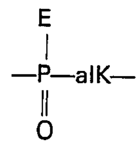

dans lesquels E peut avoir les mêmes significations que X, ou un radical —alK—O—alK—, ou un radical

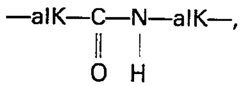, ou un radical 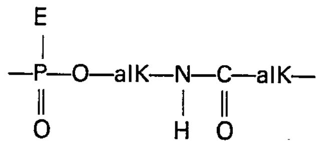 ou un radical 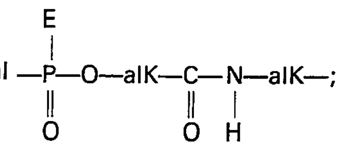;

Z et Z′ représentent chacun un radical intercalant ou un radical porteur d'au moins une fonction, réagissant directement ou indirectement avec les chaînes de nucléotides, l'un des R ou R′ pouvant représenter H lorsque le radical chimique réactif est également un radical intercalant; sous réserve que le composé A comporte au moins un radical intercalant et un radical chimique réactif, dans le cas où Z ou Z′ représente un radical intercalant qui est également un radical chimique réactif, alors Z et Z′ peuvent être identiques ou bien l'un des R ou R′ peut représenter l'hydrogène;

L représente un atome d'oxygène, un atome de soufre ou un groupe —NH—;

J représente un atome d'hydrogène ou un groupe hydroxy;

n est un nombre entier y compris O.

3. Procédé de préparation de composés selon la revendication 2, caractérisé en ce que le radical intercalant est dérivé des composés polycycliques ayant une configuration plane.

4. Procédé de préparation de composés selon la revendication 3, caractérisé en ce que le radical intercalant est dérivé de l'acridine, la furocoumarine, la daunomycine, la 1,10-phénanthroline, le phénanthridinium, les porphyrines, les dérivés de la dipyrido (1,2-a: 3′,2′-d) imidazole, l'ellipticine ou l'ellipticinium et les dérivés de ces agents intercalants.

5. Procédé de préparation de composés selon les revendications 1 à 4, caractérisé en ce que les groupes chimiques réactifs sont choisis parmi les dérivés de l'acide éthylènediamine-tétraacétique, de l'acide diéthylènetriamine-pentacétique, des porphyrines, de la 1,10-phénanthroline.

6. Procédé de préparation de composés selon l'une des revendications 1 à 5, caractérisé en ce que le radical B est un radical correspondant à la thymine, l'adénine, la 2-aminoadénine et ses dérivés substitués, par exemple sur le $N^6$, par un groupe aminoalkylène ou par un groupe azidophénylalkylène, la cytosine, la guanine ou la guanine substituée sur le $O^6$, par exemple par un groupe (ω-alkylène)-9-acridine; ou la 8-(ω-aminoalkyl)-aminoadénine et ses dérivés substitués sur le $NH_2$ en ω par un groupe acridine; l'uracile, le 5-bromo-uracile, le 8-azido-adénine ou un dérivé photoactivable des bases.

7. Procédé de préparation de composés selon la revendication 1 de formule choisie parmi:

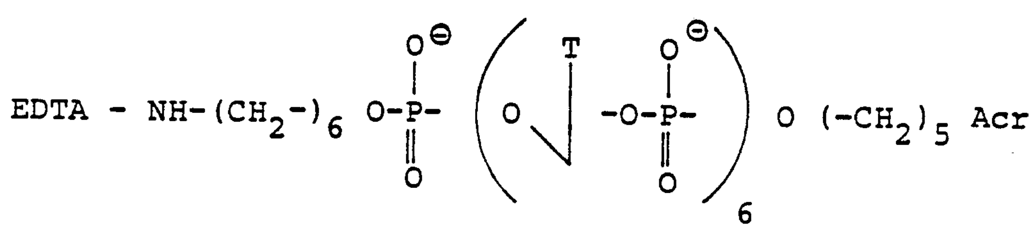

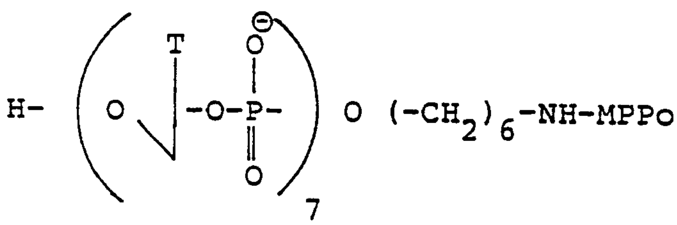

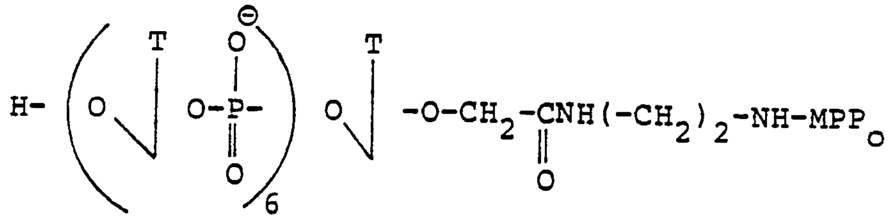

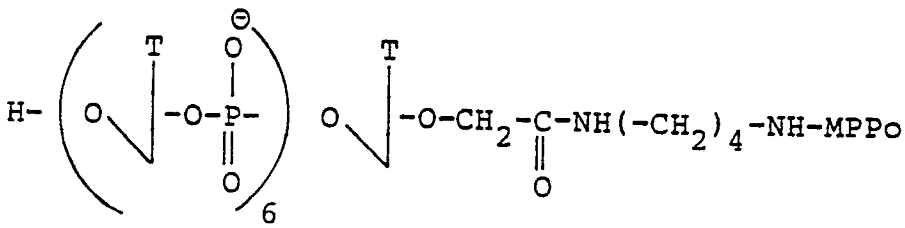

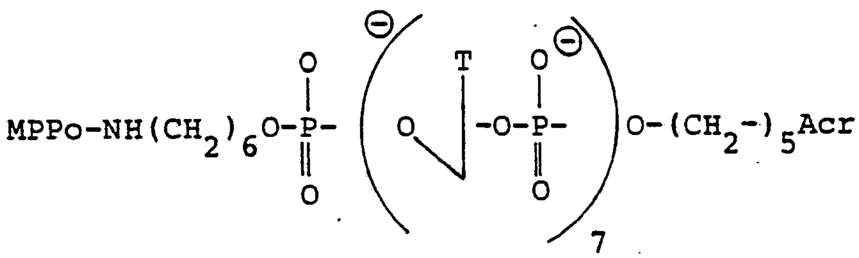

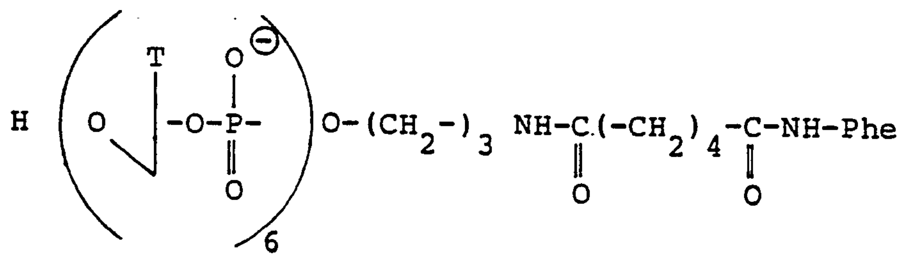

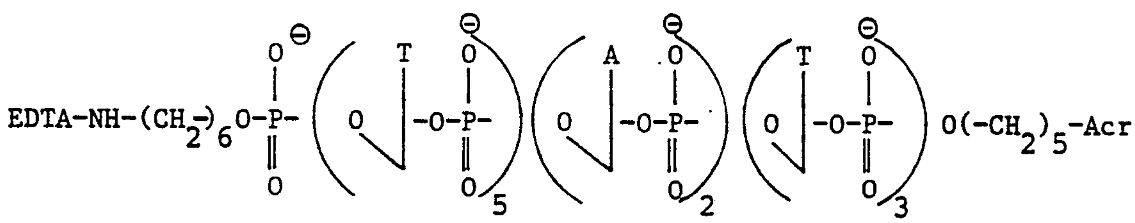

8. Utilisation de composés selon l'une des revendications 1 à 7 pour la préparation de nucléases artificielles spécifiques de séquences d'acides ribo et désoxyribonucléiques.

9. Utilisation de composés selon l'une des revendications 1 à 7 pour préparer des fragments d'acides nucléiques déterminés à partir d'acides ribo et désoxyribonucléiques.

10. Utilisation de composés selon l'une des revendications 1 à 7 pour préparer des réactifs pour analyser les structures secondaires et tertiaires d'acides ribo et désoxyribonucléiques, par coupures sélectives de la séquence cible et des séquences voisines dans l'espace.

11. Utilisation de composés selon l'une des revendications 1 à 7 pour la préparation d'agents utiles pour couper et dégrader sélectivement les acides nucléiques en particulier les ARN messagers in vivo.

12. Utilisation selon la revendication 11, pour la préparation d'agents utiles pour bloquer sélectivement l'expression d'un gène par coupure sélective du gène ou de son ARN messager.

13. Utilisation de composés selon l'une des revendications 1 à 7 à la préparation de médicament.

**Patentansprüche für die Vertragsstaaten: BE CH DE FR GB IT LI LU NL SE**

1. Chemische Verbindungen, bestehend aus einem Oligonucleotid oder einem Oligodesoxynucleotid, das eine Kette von natürlichen oder modifizierten Nucleotiden umfaßt und durch eine kovalente Bindung an einen Interkalierungsrest und an einen chemisch reaktiven Rest oder an einen Interkalierungsrest, der gleichzeitig ein chemisch reaktiver Rest ist, gebunden ist.

2. Verbindungen nach Anspruch 1 der Formel

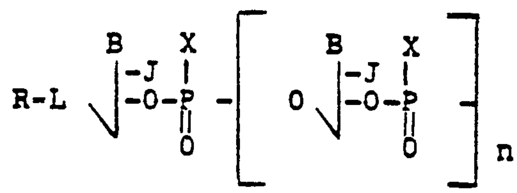 (A)

worin bedeuten:

die Reste B, die gleich oder verschieden sein können, jeweils eine natürliche oder modifizierte Nucleinsäure-Base; die Reste X, die gleich oder verscheiden sein können, jeweils ein Oxoanion $O^{\ominus}$, ein Thioanion $S^{\ominus}$, eine Alkylgruppe, eine Alkoxygruppe, Aryloxy, Alkoxy oder Alkyl, das substituiert ist durch einen stickstoffhaltigen Heterocyclus, eine Aminoalkylgruppe, eine Aminoalkoxygruppe, eine Thioalkylgruppe oder eine Gruppe der Formel —Y—Z;

R und R′, die gleich oder verschieden sein können, jeweils eine Gruppe der Formel —Y—Z oder eine Gruppe der Formel —Y′—Z′, worin Y und Y′ einen geraden oder verzweigten Alkylenrest-alK— oder die Reste

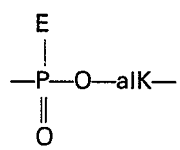 und 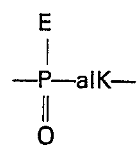

in denen E die gleichen Bedeutungen wie X haben kann, oder den Rest —alK—O—alK—, oder den Rest

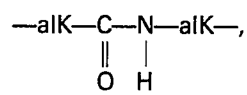, oder einen Rest 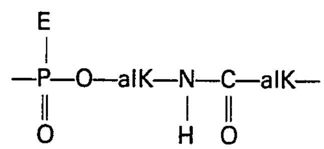 oder einen Rest

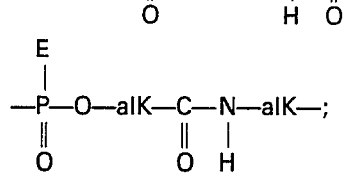

und Z und Z′ jeweils einen Interkalierungsrest oder einen Rest, der mindestens eine Funktion trägt, die direkt oder indirekt mit den Nucleotid-Ketten reagiert, darstellen, wobei einer der Reste R und R′ H darstellen kann, wenn der chemisch reaktive Rest ebenfalls ein Interkalierungsrest ist; mit der Maßgabe, daß die Verbindung A mindestens einen Interkalierungsrest und einen chemisch reaktiven Rest trägt wobei für den Fall, daß Z oder Z′ einen Interkalierungsrest darstellt, der gleichzeitig ein chemisch reaktiver Rest ist, dann Z und Z′ identisch sein können, oder auch einer der Reste R und R′ Wasserstoff darstellen kann;

L ein Sauerstoffatom, ein Schwefelatom oder eine Gruppe —NH—;

J ein Wasserstoffatom oder eine Hydroxygruppe und

n eine ganze Zahl einschließlich 0.

3. Verbindungen nach Anspruch 2, dadurch gekennzeichnet, daß der Interkalierungsrest abgeleitet ist von polycyclischen Verbindungen mit einer ebenen Konfiguration.

4. Verbindungen nach Anspruch 3, dadurch gekennzeichnet, daß der Interkalierungsrest abgeleitet ist von Acridin, Furocumarin, Daunomycin, 1,10-Phenanthrolin, Phenanthridinium, den Porphyrinen, den Derivaten des Dipyrido(1,2-a; 3′,2′-d)-imidazoles, Ellipticin oder Ellipticinium und den Derivaten dieser Interkalierungsmittel.

5. Verbindungen nach den Ansprüchen 1 bis 4, dadurch gekennzeichnet, daß die chemisch reaktiven Gruppen ausgewählt werden unter den Derivaten der Ethylendiamintetraessigsäure, der Diethylentriaminpentaessigsäure, der Porphyrine und des 1,10-Phenanthrolins.

6. Verbindungen nach einem der Ansprüche 1 bis 5, dadurch gekennzeichnet, daß der Rest B ein Rest ist, der entspricht dem Thymin, dem Adenin, dem 2-Aminoadenin und seinen Derivaten, die beispielsweise an dem $N^6$-Atom substituiert sind durch eine Aminoalkylengruppe oder eine Azidophenylalkylengruppe, dem Cytosin, dem Guanin oder dem Guanin, das an dem $O^6$-Atom substituiert ist beispielsweise durch eine (ω-Alkylen)-9-acridin-Gruppe, oder dem 8-(ω-Aminoalkyl)aminoadenin und seinen Derivaten, die an der

$NH_2$-Gruppe in ω-Stellung durch eine Acridingruppe substituiert sind; dem Uracil, dem 5-Bromo-uracil, dem 8-Azido-adenin oder einem durch Licht aktivierbaren Derivat der Basen.

7. Verbindungen nach Anspruch 1 der Formel, die ausgewählt wird aus:

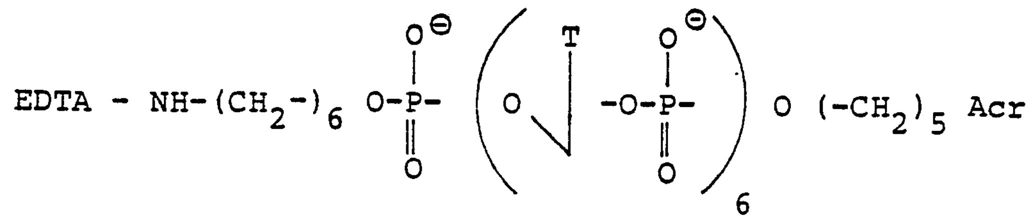

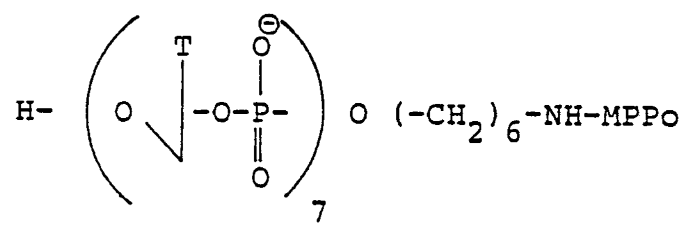

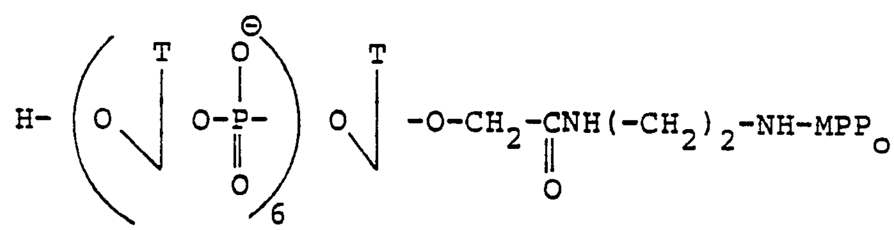

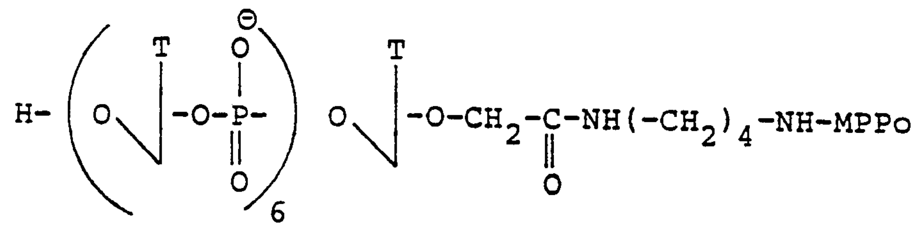

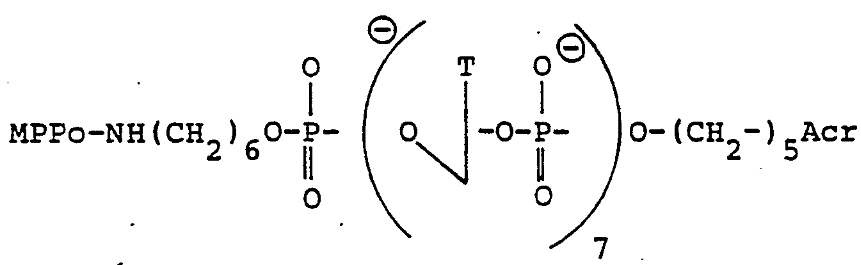

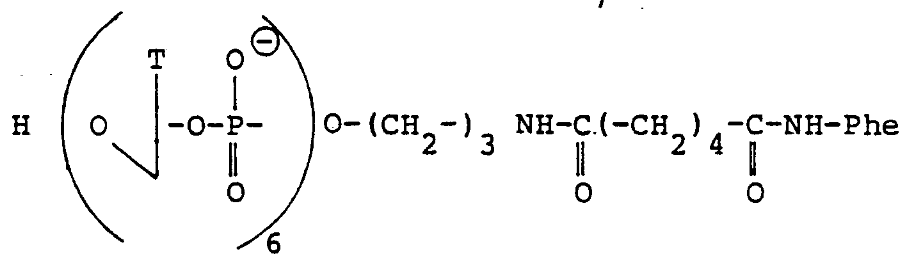

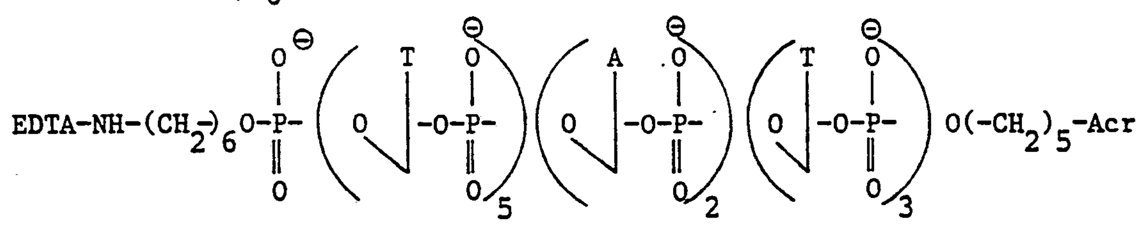

8. Verfahren zur Herstellung von Verbindungen nach einem der Ansprüche 1 bis 7, dadurch gekennzeichnet, daß man die vollständig geschützten Derivate herstellt und die Schutzgruppen eliminiert.

9. Verwendung der Verbindungen nach einem der Ansprüche 1 bis 7 als artifizielle Sequenz-spezifische Nucleasen von Ribo- und Desoxyribonucleinsäuren.

10. Verwendung der Verbindungen nach einem der Ansprüche 1 bis 7 zur Herstellung von festgelegten Nucleinsäure-Fragmenten aus Ribo- und Desoxyribonucleinsäuren.

11. Verwendung der Verbindungen nach einem der Ansprüche 1 bis 7 als Reagentien zum Analysieren der sekundären und tertiären Strukturen von Ribo- und Desoxyribonucleinsäuren durch selektive Schnitte der Zielfrequenz und der benachbarten Sequenzen im Abstand dazu.

12. Verwendung der Verbindungen nach einem der Ansprüche 1 bis 7 zum selektiven Schneiden und Abbauen der Nucleinsäuren, insbesondere der Messenger-RNA in vivo.

13. Verwendung nach Anspruch 12 zum selektiven Blockieren der Expression eines Gens durch selektives Schneiden des Gens oder seiner Messenger-RNA.

14. Verbindung nach einem der Ansprüche 1 bis 7 als Arzneimittel.

**Patentansprüche für den Vertragsstaat: AT**

1. Verfahren zur Herstellung von chemischen Verbindungen, bestehend aus einem Oligonucleotid oder einem Oligodesoxynucleotid, das umfaßt eine Kette von natürlichen oder modifizierten Nucleotiden und durch eine kovalente Bindung an einen Interkalierungsrest und an einen chemisch reaktiven Rest oder an einen Interkalierungsrest, der gleichzeitig ein chemisch reaktiver Rest ist, gebunden ist, dadurch gekennzeichnet, daß man vollständig geschützte Derivate herstellt und die Schutzgruppen eliminiert.

2. Verfahren zur Herstellung von Verbindungen nach Anspruch 1 der Formel

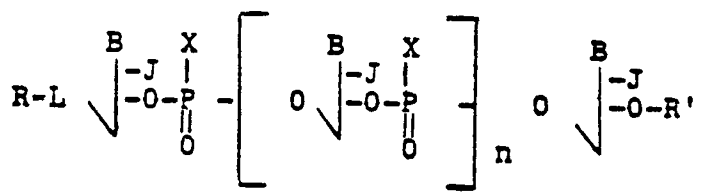

(A)

worin bedeuten:

die Reste B, die gleich oder verschieden sein können, jeweils eine natürliche oder modifizierte Nucleinsäurebase; die Reste X, die gleich oder verschieden sein können, jeweils ein Oxoanion $O^{\ominus}$, ein Thioanion $S^{\ominus}$, eine Alkylgruppe, eine Alkoxygruppe, Aryloxy, Alkoxy oder Alkyl, das durch einen stickstoffhaltigen Heterocyclus substituiert ist, eine Aminoalkylgruppe, eine Aminoalkoxygruppe, eine Thioalkylgruppe oder eine Gruppe der Formel —Y—Z;

R und R', die gleich oder verschieden sein können, jeweils eine Gruppe der Formel —Y—Z oder eine Gruppe der Formel —Y'—Z', worin Y und Y' einen geraden oder verzweigten Alkylenrest-alK— oder die Reste

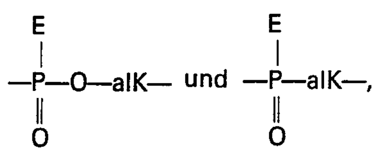

worin E die gleichen Bedeutungen wie X haben kann, oder einen Rest —alK—O—alK—, oder einen Rest

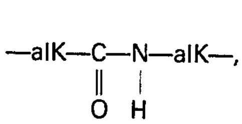

oder einen Rest

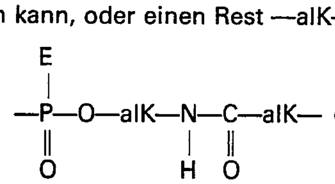

oder einen Rest

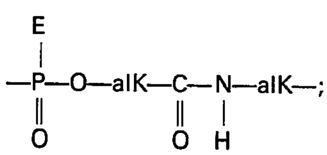

und Z und Z' jeweils einen Interkalierungsrest oder einen Rest, der mindestens eine Funktion trägt, die direkt oder indirekt mit den Nucleotid-Ketten reagiert, darstellen, wobei einer der Reste R und R' H darstellen kann, wenn der chemisch reaktive Rest gleichzeitig ein Interkalierungsrest ist; mit der Maßgabe, daß die Verbindung A mindestens einen Kalierungsrest und einen chemisch reaktiven Rest aufweist, wobei für den Fall, daß Z oder Z' einen Interkalierungsrest darstellt, der gleichzeitig ein chemisch reaktiver Rest

ist, dann Z und Z' identisch sein können, oder auch einer der Reste R und R' Wasserstoff darstellen kann;

L ein Sauerstoffatom, ein Schwefelatom oder eine Gruppe —NH—;

J ein Wasserstoffatom oder eine Hydroxygruppe und

n eine ganze Zahl einschließlich 0.

3. Verfahren zur Herstellung von Verbindungen nach Anspruch 2, dadurch gekennzeichnet, daß der Interkalierungsrest von polycyclischen Verbindungen mit einer ebenen Konfiguration abgeleitet ist.

4. Verfahren zur Herstellung von Verbindungen nach Anspruch 3, dadurch gekennzeichnet, daß der Interkalierungsrest von Acridin, Furocumarin, Daunomycin, 1,10-Phenanthrolin, Phenanthridinium, den Porphyrinen, den Derivaten des Dipyrido(1,2-a; 3',2'-d)imidazols, Ellipticin oder Ellipticinium und den Derivaten dieser Interkalierungsmittel abgeleitet ist.

5. Verfahren zur Herstellung von Verbindungen nach den Ansprüchen 1 bis 4, dadurch gekennzeichnet, daß die chemisch reaktiven Gruppen ausgewählt werden aus den Derivaten der Ethylendiamintetraessigsäure, der Diethylentriaminpentaessigsäure, der Porphyrine und von 1,10-Phenanthrolin.

6. Verfahren zur Herstellung von Verbindungen nach einem der Ansprüche 1 bis 5, dadurch gekennzeichnet, daß der Rest B ein Rest ist, der entspricht dem Thymin, dem Adenin, dem 2-Aminoadenin und seinen Derivaten, die beispielsweise an dem $N^6$-Atom substituiert sind durch eine Aminoalkylen- oder eine Azidophenylalkylen-Gruppe, dem Cytosin, dem Guanin oder dem Guanin, das an dem $O^6$-Atom beispielsweise durch eine (ω-Alkylen)-9-acridin-Gruppe substituiert ist, oder dem 8-(ω-Aminoalkyl)aminoadenin und seinen Derivaten, die an der $NH_2$-Gruppe in ω-Stellung durch eine Acridingruppe substituiert sind; dem Uracil, dem 5-Bromo-uracil, dem 8-Azido-adenin oder einem durch Licht aktivierbaren Derivat der Basen.

7. Verfahren zur Herstellung von Verbindungen nach Anspruch 1 der Formel, die ausgewählt wird aus

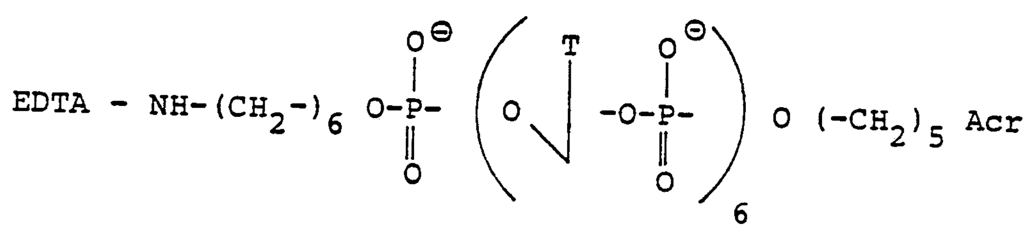

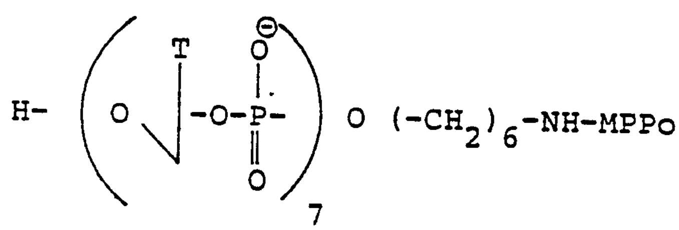

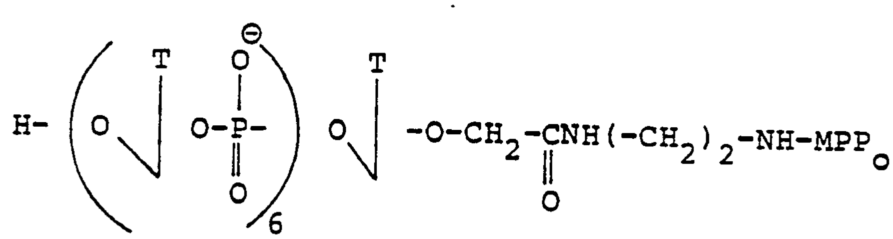

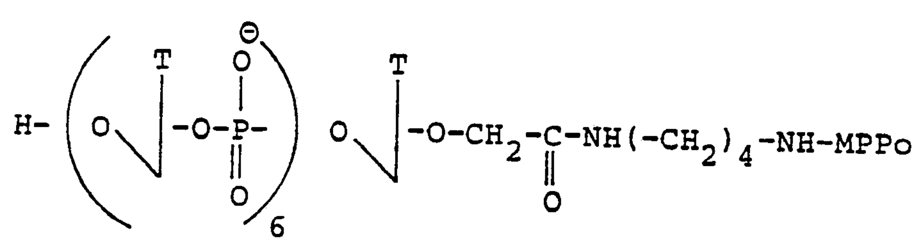

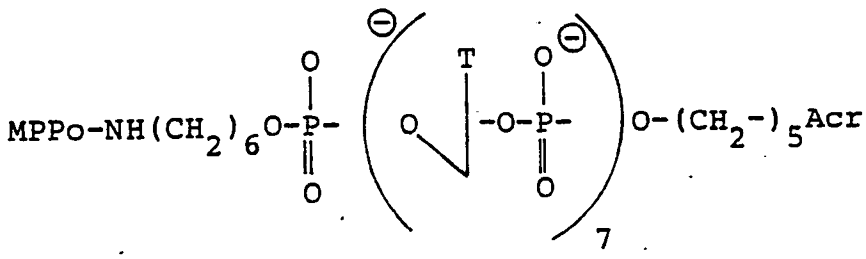

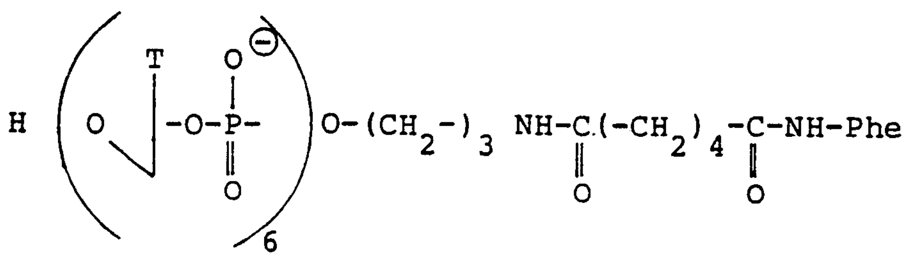

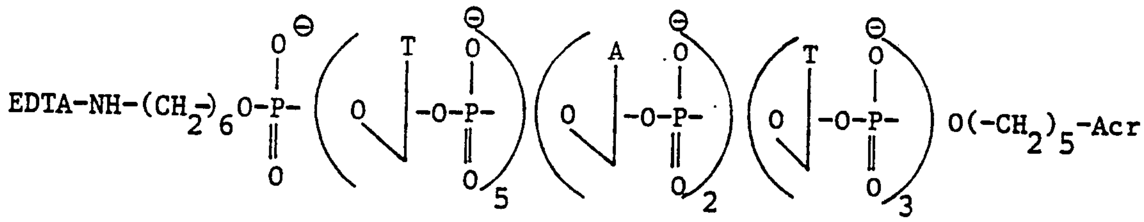

8. Verwendung von Verbindungen nach einem der Ansprüche 1 bis 7 zur Herstellung von artifiziellen Sequenz-spezifischen Nucleasen von Ribo- und Desoxyribonucleinsäuren.

9. Verwendung der Verbindungen nach einem der Ansprüche 1 bis 7 zur Herstellung von vorgegebenen Nucleinsäure-Fragmenten aus Ribo- und Desoxyribonucleinsäuren.

10. Verwendung der Verbindungen nach einem der Ansprüche 1 bis 7 zur Herstellung von Reagentien zum Analysieren der sekundären und tertiären Strukturen von Ribo- und Desoxyribonucleinsäuren durch selektive Schnitte er Zielsequenz und benachbarter Sequenzen im Abstand dazu.

11. Verwendung der Verbindungen nach einem der Ansprüche 1 bis 7 zur Herstellung von Agentien, die zum Schneiden und selektiven Abbauen der Nucleinsäuren, insbesondere der Messenger-RNA in vivo geeignet sind.

12. Verwendung nach Anspruch 11 zur Herstellung von Agentien, die zum selektiven Blockieren der Expression eines Gens durch selektiven Schnitt des Gens oder seiner Messenger-RNA geeignet sind.

13. Verwendung von Verbindungen nach einem der Ansprüche 1 bis 7 zur Herstellung eines Arzneimittels.

**Claims for the Contracting States: BE CH DE FR GB IT LI LU NL SE**

1. Chemical compounds consisting of an oligonucleotide or an oligodeoxynucleotide containing an arrangement of natural or modified nucleotides and linked through a covalent bond to an intercalating radical and to a reactive chemical radical or to an intercalating radical which is also a reactive chemical radical.

2. Compounds as claimed in claim 1, of formula:

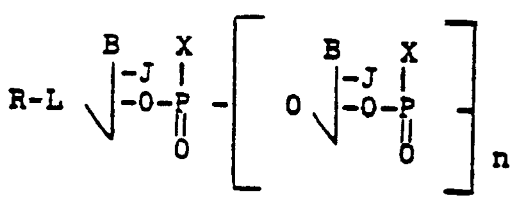

(A)

in which:

the radicals B can be identical or different and each denote a nucleic acid base, natural or modified;

the radicals X, which can be identical or different, each denote an oxo anion $O^-$, a thio anion $S^-$, an

alkyl group, an alkoxy or aryloxy group, an alkoxy or alkyl group substituted with a nitrogen-containing heterocyclic system, an aminoalkyl group, an aminoalkoxy group, a thioalkyl group or a group —Y—Z;

R and R′ can be identical or different and each denote a group —Y—Z or a group —Y′—Z′ in which Y and Y′ denote a linear or branched alkylene radical —alK—, or

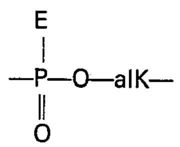 and 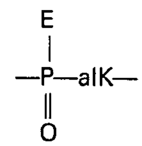

in which E can have the same meanings as X, or a radical —alK—O—alK—, or a radical

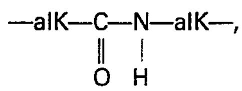, or a radical 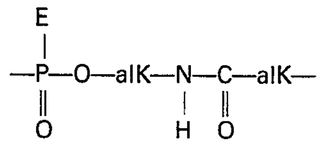 or a radical 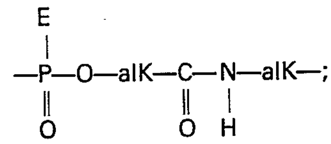

Z and Z′ each denote an intercalating radical or a radical bearing at least one group which reacts directly or indirectly with nucleotide chains, it being possible for one of R or R′ to denote H when the reactive chemical radical is also an intercalating radical; with the proviso that the compound A contains at least one intercalating radical and a reactive chemical radical, in the case where Z or Z′ denotes an intercalating radical which is also a reactive chemical radical, then Z and Z′ can be identical or alternatively one of R or R′ can denote hydrogen;

L denotes an oxygen atom, a sulfur atom or an —NH— group;

J denotes a hydrogen atom or a hydroxy group;

n is an integer including 0.

3. Compounds as claimed in claim 2, in which the intercalating radical is derived from polycyclic compounds having a plane configuration.

4. Compounds as claimed in claim 3, in which the intercalating radical is derived from acridine, furocoumarin, daunomycin, 1,10-phenanthroline, phenanthridinium porphyrins, dipyrido[1,2-a: 3′,2′-d]imidazole derivatives and ellipticine or ellipticinium and derivatives of these intercalating agents.

5. Compounds as claimed in claims 1 to 4, in which the reactive chemical groups are chosen from derivatives of ethylenediaminetetraacetic acid, of diethylenetriaminepentaacetic acid, of porphyrins and of 1,10-phenanthroline.

6. Compounds as claimed in one of claims 1 to 5, in which the radical B is a radical corresponding to thymine, adenine, 2-aminoadenine and its derivatives substituted, for example, on $N^6$ with an aminoalkylene group or with an azidophenylalkylene group, cytosine, guanine or guanine substituted on $O^6$, for example, with a 9-(ω-alkylene)acridine group; or 8-(ω-aminoalkyl)aminoadenine and its derivatives substituted on the ω-$NH_2$ with an acridine group; uracil, 5-bromouracil, 8-azidoadenine or a derivative of the bases which can be photoactivated.

7. Compounds as claimed in claim 1, of formula chosen from:

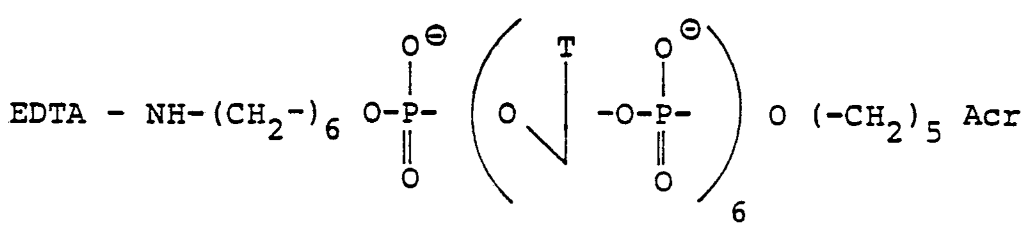

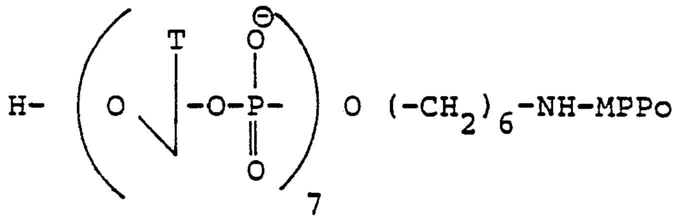

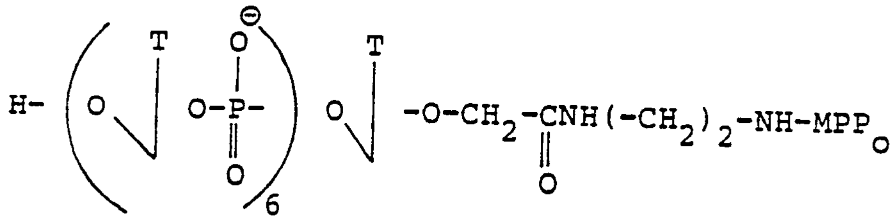

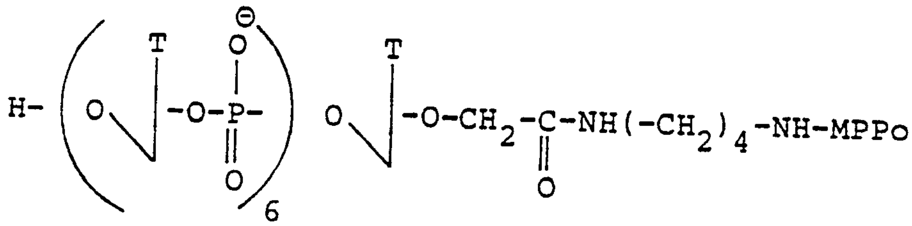

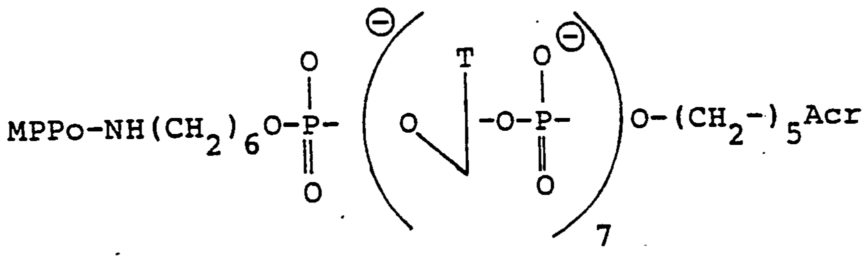

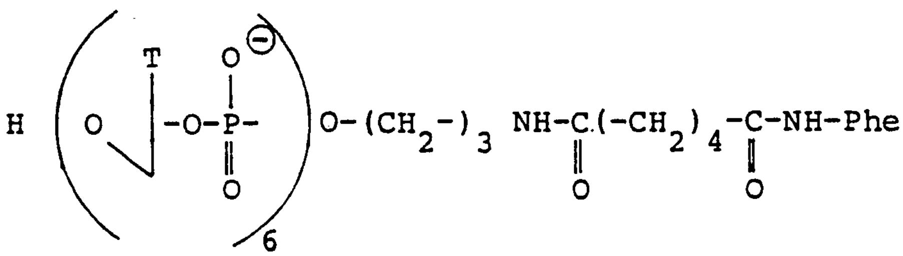

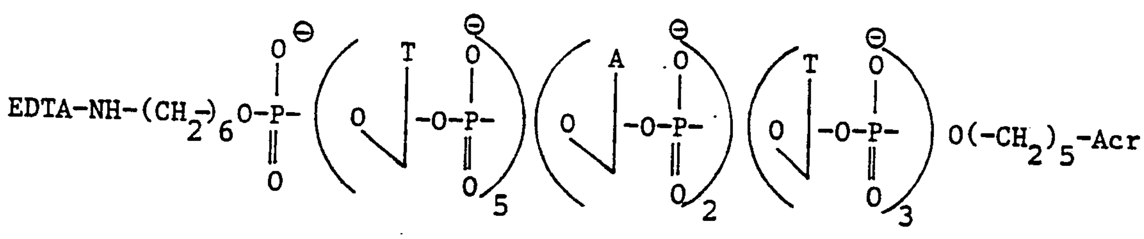

8. A process for preparing compounds as claimed in one of claims 1 to 7, wherein the fully protected derivatives are prepared and wherein the protective groups are removed.

9. Application of the compounds as.claimed in one of claims to 1 to 7 by way of artificial nucleases specific for ribo- and deoxyribonucleic acid sequences.

10. Application of the compounds as claimed in one of claims 1 to 7 for obtaining specified nucleic acid fragments from ribo- and deoxyribonucleic acids.

11. Application of the compounds as claimed in one of claims 1 to 7 as reagents for analyzing the secondary and tertiary structures of ribo- and deoxyribonucleic acids, by selective cleavage of the target sequence and of the sequences in spatial proximity.

12. Application of the compounds as claimed in one of claims 1 to 7 for selectively cleaving and degrading nucleic acids, especially messenger RNAs in vivo.

13. The application as claimed in claim 12, for selectively blocking the expression of a gene by selective cleavage of the gene or of its messenger RNA.

14. By way of a drug, the compound as claimed in one of claims 1 to 7.

**Claims for the Contracting State: AT**

1. Preparation process of chemical compounds consisting of an oligonucleotide or an oligodeoxynucleotide containing an arrangement of natural or modified nucleotides and linked through a covalent bond to an intercalating radical and to a reactive chemical radical or to an intercalating radical which is also a reactive chemical radical wherein the fully protected derivatives are prepared and the protecting groups are removed thereafter.

2. Preparation process of compounds as claimed in claim 1, of formula:

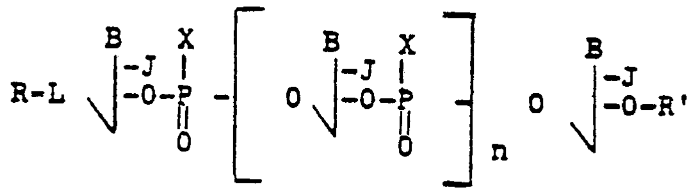
(A)

in which:

the radicals B can be identical or different and each denote a nucleic acid base, natural or modified;

the radicals X, which can be identical or different, each denote an oxo anion $O^-$, a thio anion $S^-$, an alkyl group, an alkoxy or aryloxy group, an alkoxy or alkyl group substituted with a nitrogen-containing heterocyclic system, an aminoalkyl group, an aminoalkoxy group, a thioalkyl group or a group —Y—Z;

R and R′ can be identical or different and each denote a group —Y—Z or a group —Y′—Z′ in which Y and Y′ denote a linear or branched alkylene radical —alK—, or

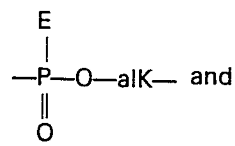
and
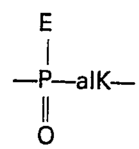

in which E can have the same meanings as X, or a radical —alK—O—alK—, or a radical

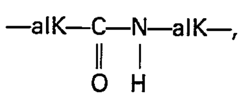
, or a radical
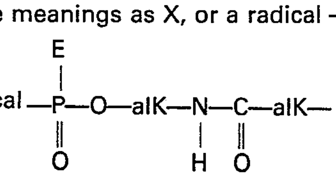
or a radical
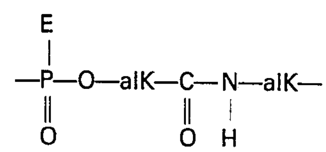

Z and Z′ each denote an intercalating radical or a radical bearing at least one group which reacts directly or indirectly with nucleotide chains, it being possible for one of R or R′ to denote H when the reactive chemical radical is also an intercalating radical; with the proviso that the compound A contains at least one intercalating radical and a reactive chemical radical, in the case where Z or Z′ denotes an intercalating radical which is also a reactive chemical radical, then Z and Z′ can be identical or alternatively one of R or R′ can denote hydrogen;

L denotes an oxygen atom, a sulfur atom or an —NH— group;

J denotes a hydrogen atom or a hydroxy group;

n is an integer including 0.

3. Preparation process of compounds as claimed in claim 2, in which the intercalating radical is derived from polycyclic compounds having a plane configuration.

4. Preparation process of compounds as claimed in claim 3, in which the intercalating radical is derived from acridine, furocoumarin, daunomycin, 1,10-phenanthroline, phenanthridinium porphyrins, dipyrido[1,2-a: 3′,2′-d]imidazole derivatives and ellipticine or ellipticinium and derivatives of these intercalating agents.

5. Preparation process of compounds as claimed in claims 1 to 4, in which the reactive chemical groups are chosen from derivatives of ethylenediaminetetraacetic acid, of diethylenetriamine-pentaacetic acid, of porphyrins and of 1,10-phenanthroline.

6. Preparation process of compounds as claimed in one of claims 1 to 5, in which the radical B is a radical corresponding to thymine, adenine, 2-aminoadenine and its derivatives substituted, for example, on $N^6$ with an aminoalkylene group or with an azidophenylalkylene group, cytosine, guanine or guanine substituted on $O^6$, for example, with 9-(ω-alkylene)acridine group; or 8-(ω-aminoalkyl)aminoadenine and its derivatives substituted on the $\omega$-$NH_2$ with an acridine group; uracil, 5-bromouracil, 8-azidoadenine or a derivative of the bases which can be photoactivated.

7. Preparation process of compounds as claimed in claim 1, of formula chosen from:

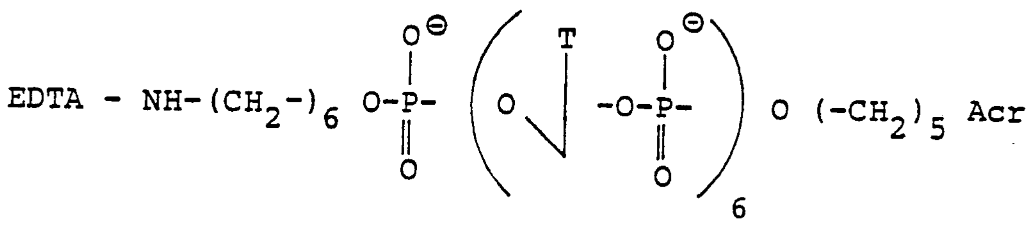

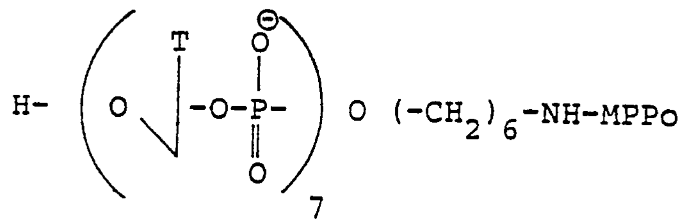

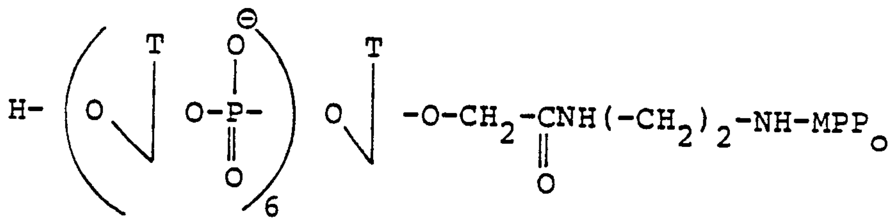

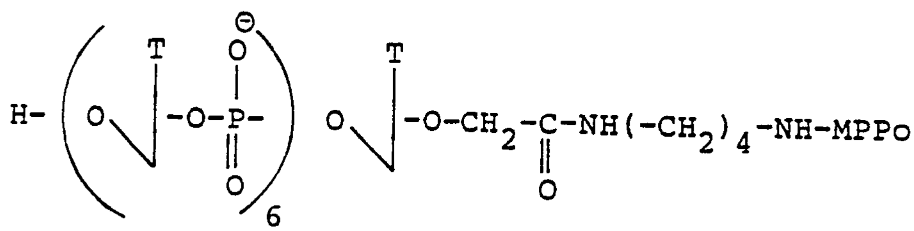

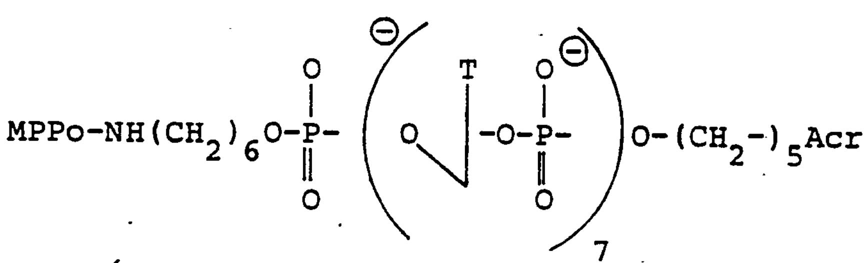

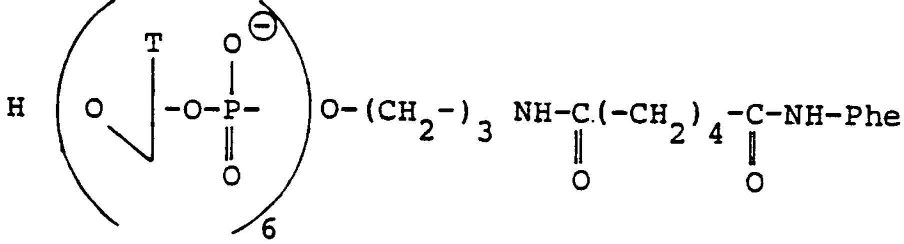

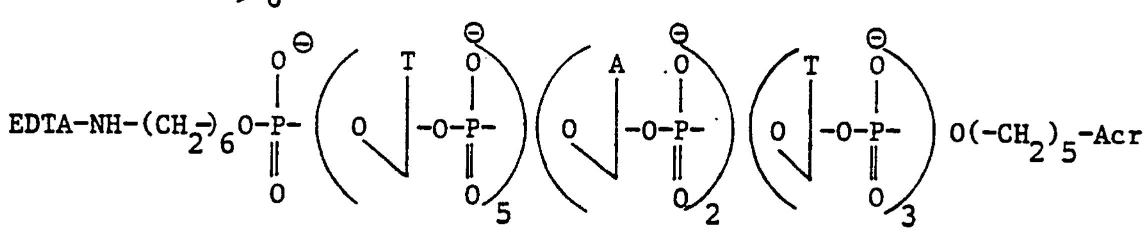

8. Application of the compounds as claimed in one of claims to 1 to 7 for the preparating of artificial nucleases specific for ribo- and deoxyribonucleic acid sequences.

9. Application of the compounds as claimed in one of claims 1 to 7 for preparating specified nucleic acid fragments from ribo- and deoxyribonucleic acids.

10. Application of the compounds as claimed in one of claims 1 to 7 for preparing reagents for analyzing the secondary and tertiary structures of ribo- and deoxyribonucleic acids, by selective cleavage of the target sequence and of the sequences in spatial proximity.

11. Application of the compounds as claimed in one of claims 1 to 7 for preparing agents useful for selectively cleaving and degrading nucleic acids, especially messenger RNAs in vivo.

12. The application as claimed in claim 12, for preparing agents useful for selectively blocking the expression of a gene by selective cleavage of the gene or of its messenger RNA.

13. Application of the compounds of claims 1 to 7 for preparing drugs.

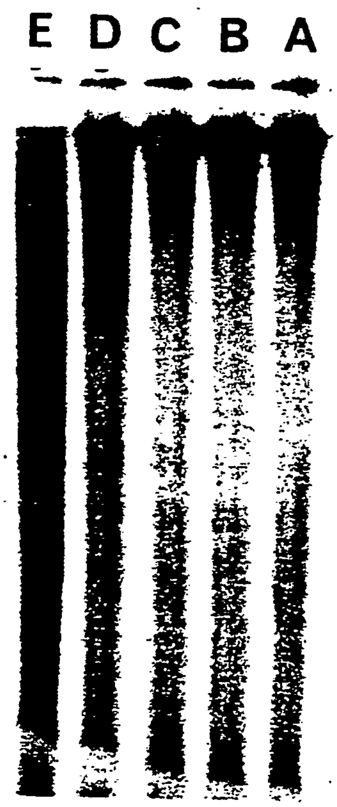
E D C B A